# EUROPEAN PATENT SPECIFICATION

(11) **EP 2 503 973 B1**
(45) Date of publication and mention of the grant of the patent: **17.02.2016**
(21) Application number: 10787351.5
(22) Date of filing: 24.11.2010
(51) Int. Cl.: A61F 13/15

(54) **SHAPED PANTS-STYLE ARTICLES AND METHOD FOR PRODUCTION**
HOSENFÖRMIGE ARTIKEL UND VERFAHREN ZUR HERSTELLUNG
ARTICLE DE TYPE CULOTTE ET PROCÉDÉ DE FABRICATION

(30) Priority: 24.11.2009 GB 0920571; 22.03.2010 GB 201004733; 01.10.2010 GB 201016540
(43) Date of publication of application: 03.10.2012
(73) Proprietor: Concepts For Success (C4S), 53881 Euskirchen-Stotzheim (DE)
(72) Inventor: SCHMITZ, Christoph, D-53881 Euskirchen-Stotzheim (DE)
(74) Representative: Plischke, Manfred
(86) International application number: PCT/EP2010/068162
(87) International publication number: WO 2011/064275

(56) References cited:
- EP-A1- 0 901 780
- WO-A1-2007/123445
- WO-A2-02/36060
- US-A1- 2002 138 062
- US-A1- 2005 222 549
- US-A1- 2006 241 560

## Description

### Field of the invention

The present invention is an article such as a garment to be worn on the lower torso of a wearer, such as pants or diapers. The article exhibits snug fit around the legs of a wearer through a particular cooperative arrangement of side panels and a centre strip. The present invention is further a manufacturing method for producing such articles.

### Background

Because of the quite complex shape of human bodies, the automated manufacturing of garments is typically a compromise of manufacturing speed versus complexity of the shape of the garment or article.

On one side, very slow machines, which may include sewing and /or even manual intervention for certain steps, can produce quite complex garments such as well fitting pants or underwear, such as described in US7155940 in the context sewing three knitted half-blanks into a panty garment.

The other extreme are for example high speed machines producing disposable articles, such as baby or adult incontinence diapers, training pants and the like at production speeds of several hundred articles per minute or even more. Such articles, however, are much less complex in their design with regard to shape, and whilst the body fit is often somewhat improved by elastic materials, the manufacturing costs are significantly increased. Typically, such articles are manufactured from two-dimensional webs, such as nonwoven or films, by merely overlaying, connecting, and folding. In the early development of disposable diapers, such articles have been manufactured by folding the longitudinal outer side portions of an essentially rectangular web or pad over the mid portion, either as a simple overfold, such as e.g. described in US3572342, or as a double pleat (Z-fold) such as described in US3426756. In the crotch region, the fold was secured by a so called "centre glue", such that upon use the pad was unfolded in the front and/or rear waist region, but it retained a narrow configuration in the crotch region, thereby approximating an hourglass shape. Further developments introduced the "shaped diapers" by introducing leg cut outs, elastic elements, waist ears etc., etc. Out of the plethora of publications in this area, WO08/118713, WO98/14156, EP0901780A1 may serve as examples showing such contemporary conventional articles.

Recently, "body conforming articles" as well as manufacturing methods for such articles have been described such as in WO06/102974.

Whilst these articles show an improved body fit, particularly in the leg and crotch area, there is still a need for increasing the flexibility in the design and in particular in the material selection and arrangement in the article.

There is also a need for alternative production methods, in particular with regard to adapting existing manufacturing equipment. There is also a need for increased output per converting line.

### Brief description of the Figures

Fig. 1 shows an article in the form of a pants style absorbent article.
Fig. 2A to C show schematically an article according to the present invention.
Fig. 3 shows schematically a particular embodiment of the present invention.
Fig. 4 shows schematically a further particular feature of the present invention with regard to connecting regions.
Fig. 5 shows schematically an article comprising unitary side panel and centre as a further particular execution according to the present invention.
Fig. 6 shows schematically an article comprising legs hoops as yet a further particular execution of the present invention.
Fig. 7 shows schematically an article wherein the side panel is folded and glued so as to provide the functionality of a leg hoop.
Fig. 8 shows schematically further embodiments according to the present invention.
Fig. 9A to E show various executions according to the present invention relating to the double pleats, with Fig. 9 (1), (2), (5), (6), (9), (11), (14), (27), (28), (29), (30), and (31) representing the state of the art.
Fig. 10 shows schematically an article according to the present invention in a pre-use configuration.
Fig. 11 A to G show schematically the process steps for forming articles comprising leg hoops in an MD-manufacturing process.
Fig. 12A to D show schematically the process aspects for forming closed articles.
Fig. 13A and B show schematically a setup of a CD- and MD- multi-lane converter,

The same numerals across different figures denote identical features

The present invention relates to articles, typically worn by humans, although not necessarily limited thereto. Within the context of the present invention, the term "article" shall also comprise "pre-cursor of an article", such as when an unfinished precursor, which is made according to the process described herein is finished such as by adding further elements or features. The articles according to the present invention are preferably shaped so as to adapt well to body contours in particular around the lower torso and around the legs in the upper thigh respectively crotch region. Within the present context, the leg region of a standing wearer extends from the crotch line downwards, whereby the crotch line is defined by the crotch crease or groin adjacent to the pubic area and an essentially horizontal circumferential line through the groin laterally outwardly around the leg.

The articles according to the present invention comprise a front and a rear region, typically corresponding to the waist regions of a wearer, and a crotch region there between, thereby defining the longitudinal orientation if the article. The article further comprises side panel regions, which extend laterally outwardly of the centre piece when the article is in its in-use configuration. In a pre-use configuration, i.e. during manufacturing, or in a folded article after manufacturing, the side panels may be folded so as to or otherwise overlay the centre piece. Between the crotch region and the waist regions, the article comprises hip regions, whereby all these regions correspond to the regions of a wearer during its intended use. The articles may further comprise a left and right leg hoop, which during use encircle the upper thigh of a wearer. The article according to the present invention is essentially symmetrical to its longitudinal centreline. Nonetheless the description may refer to one major part of the article only, say the part which corresponds to the left back body parts of a wearer, but yet the features may be applied to the three other major parts of the article.

Thus, an article according to the present invention comprises a front region, a rear region and a crotch region there between, thereby defining a longitudinal (x-) and width (y-) direction and a longitudinally extending centre line of the article. A centre strip extends from the front region through the crotch region to the rear region, exhibiting a first and a second longitudinally extending side margin and a first and a second surface. The articles comprise at least a first and a second rear, optionally a first and a second front side panel connected to the centre piece in connecting regions, respectively wherein each of the side panels exhibit a first and second longitudinally extending side margin and a first and a second surface. The centre piece connecting regions extend essentially along the side margins of the centre piece in the rear region and optionally front region of the centre piece and the side panel connecting regions extend essentially along the first side margins of the side panels,

At least in the pre-use configuration, the centre piece and the side panels may be positioned such that their second surfaces are oriented towards each other and the first side margin of the first rear side panel is connected to the first side margin of the centre piece and the first) side margin of the second rear side panel is connected to the second side margin of the centre piece. Optionally the first side margin of the first front side panel is connected to the first side margin of the centre piece and the first side margin of the second front side panel is connected to the second side margin of the centre piece. Through this arrangement, the second side margins of the first and second rear and optionally front side panels extend towards and optionally beyond the longitudinally extending centre line of the centre piece, but not to the connecting region of the respective opposite side panel.

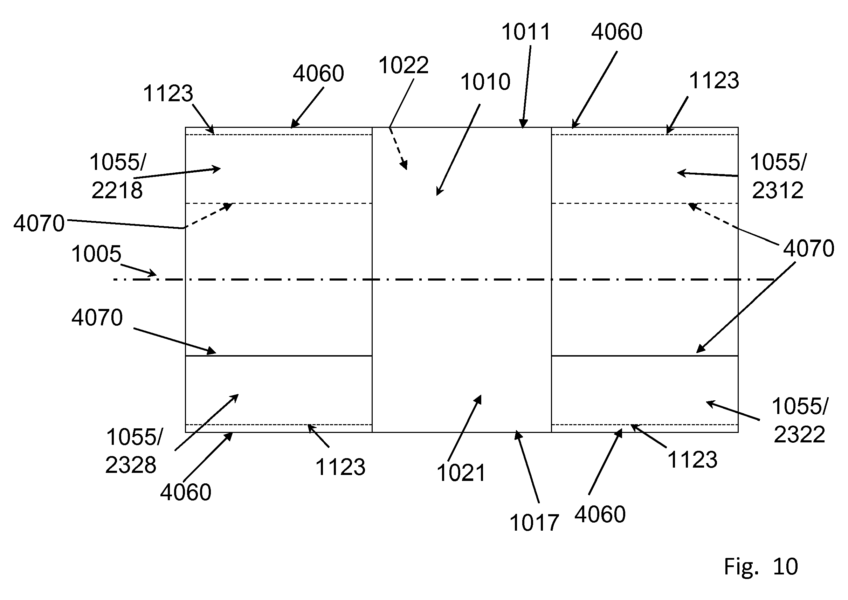

Fig. 10 shows an article respectively a pre-cursor there for according to the present invention in a pre-use configuration, having a centre piece 1010 with side panels 1055 connected thereto. Although a first (2318) and a second (2328) rear as well as a first (2312) and a second (2322) front side panel are shown, the latter ones are not necessary for the invention to function. The centre piece has a second surface, here shown as article's outer surface 1021 oriented towards the viewer and first opposite surface (1022), which will be oriented towards the user in the in-use configuration (e.g. a topsheet surface). The side panels are shown as individual web pieces connected to the centre piece in the connection regions 1123, here shown as straight lines, which may be glue lines or regions, or which may be ultrasonic or heat pressure bonding lines or regions. These connection regions extend essentially along a first (1011) and an opposite second (1017) longitudinally extending side margin of the centre piece 1010 at least in the rear region and - as shown in Fig. 10 - also in the front regions and the corresponding connecting regions of the side panels along a first longitudinally extending side margin of the side panels. The sidepanels overlay the centre piece with the second of their surfaces oriented towards the centre piece such that the facing surfaces of the centre piece and the side panel are corresponding surfaces during use, i.e. as shown in Fig. 10, the outer surface 1021 of the centre piece is oriented towards the viewer whilst the surface of the side panels oriented towards the viewer will be the inner surface oriented towards the user during use. Considering the first rear side panel 2318, the second longitudinally extending side margin 4070 is positioned away from the first longitudinally extending side margin1011 of the centre piece towards the longitudinally extending centre line 1005 or beyond, but not into, the connecting region of the second rear side panel.

Optionally, the side panels may be folded or even be multiply folded, such as in an "accordion" or "leporello" type of fold. Optionally, the sidepanels are composed of more than 1 web, connected to each other along connecting regions by butt or overlapping connections.

Such a pre-cursor can be readily converted into a finished article, such as by adding further features, such as closure tapes or by connecting the first front and rear and second front and rear side panels so as to form a pants style article, or it may already present an article except for not being in its "ready to use" or "in use" configuration.

The article may be further converted by overfolding longitudinal sections of the centre piece towards the centre line of the article such that the first (or wearer oriented) surfaces are oriented towards each other. In the crotch region, this overfold may be stabilized by connecting the surfaces to each other, such as by conventional glue bonding.

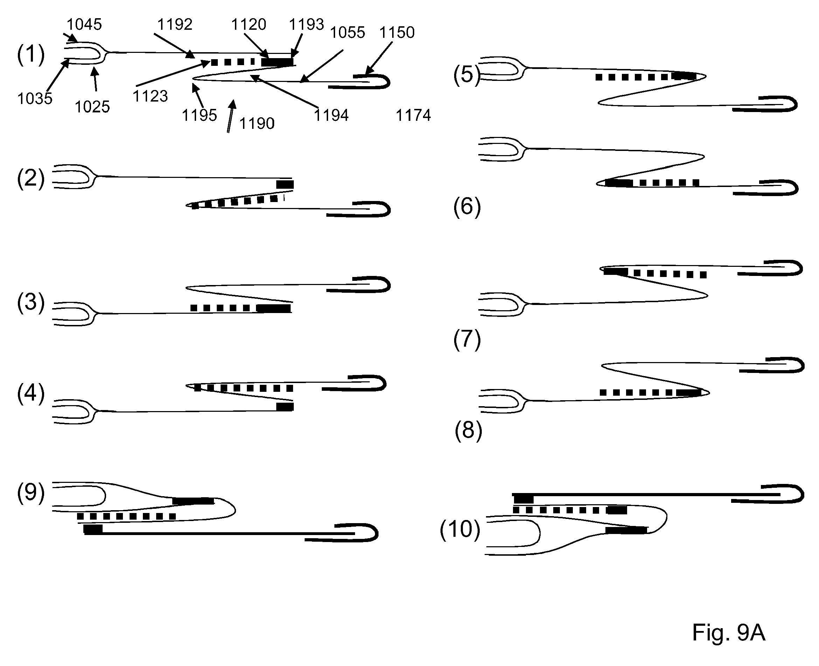

In a particular embodiment, the article can be adapted to fit particularly well to the body contours during use, which may be achieved by a particular folding of the side margins of a centre strip and laterally outwardly extending side panel regions in a double pleated or Z-fold configuration. In the making of the article at least one of the pleats has been affixed such as by connection lines or regions e.g. by adhesive or heat bonding. There are a number of options for the arrangement of the double pleats, used materials, and the affixing, as further explained with Fig. 9 A to E. Therein is exemplarily shown the embodiment of an absorbent article such as a taped diaper and one of the rear parts is depicted in a schematic cross-sectional view. As shown in Fig. 9A(1), the core 1035 is enveloped between a topsheet 1025 and a backsheet 1045, together forming the centre strip. As shown, topsheet and backsheet run as a composite into the double pleat 1190 towards the first pleat tip 1193, where they terminate. A separate side panel material 1055 has been overfolded, and the non-folded part runs laterally outwardly towards a closure element, e.g. a closure tape 1150. The longitudinal fold of the side panel forms the second pleat tip 1195, from where it further runs towards the first pleat tip where it terminates. In this first pleat 1192, the side panel is affixed to the topsheet/backsheet laminate in a butt-type attachment, such as by the first attachment 1120 (as solid line). An optional contour attachment 1123 (as dotted line), as will be described further, may be placed in the first pleat. This contour attachment may be integral with or even identical with the first attachment. The embodiment as shown in Fig. 9A(2) differs in that the optional contouring attachment is in the second pleat. Fig. 9A (3) and (4) show equivalent embodiments, except that the pleating is done in the opposite direction.

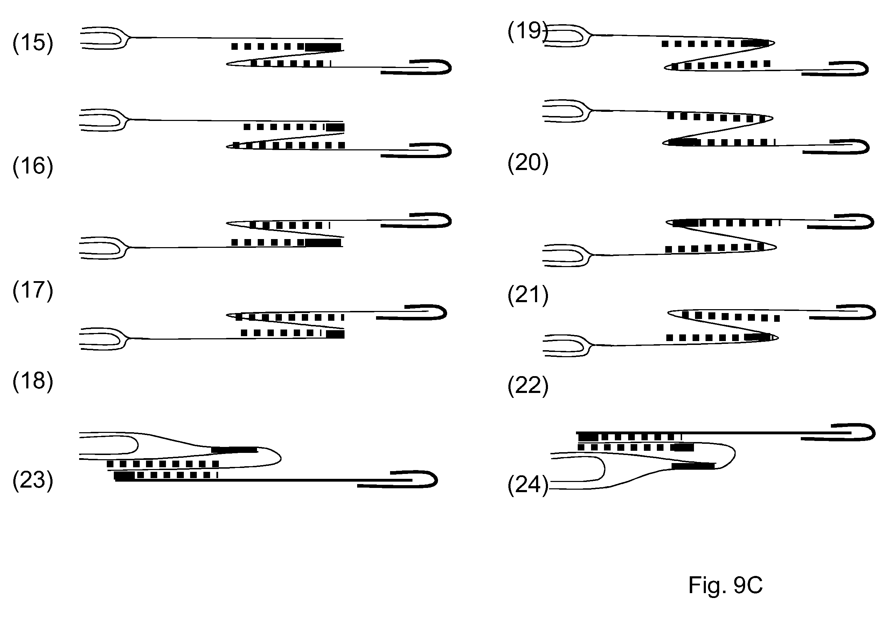

In another execution, at least one material of the centre piece may extend continuously across the centre piece and the side panel regions, such as the topsheet, or backsheet, or the topsheet/backsheet composite. Thus, as shown in Fig. 9A (5) to (8), and in analogy to Fig. 9A(1) to (4), the double pleating may be executed forward or backwardly, and the first attachment shown as solid line 1120 is positioned in the first or second pleat. The contouring attachment shown as dotted line 1123 fixation may be placed in either or both of the pleats. The contouring attachment may also be integral or identical with the first attachment 1120, as indicated with the further options depicted in Fig. 9C.

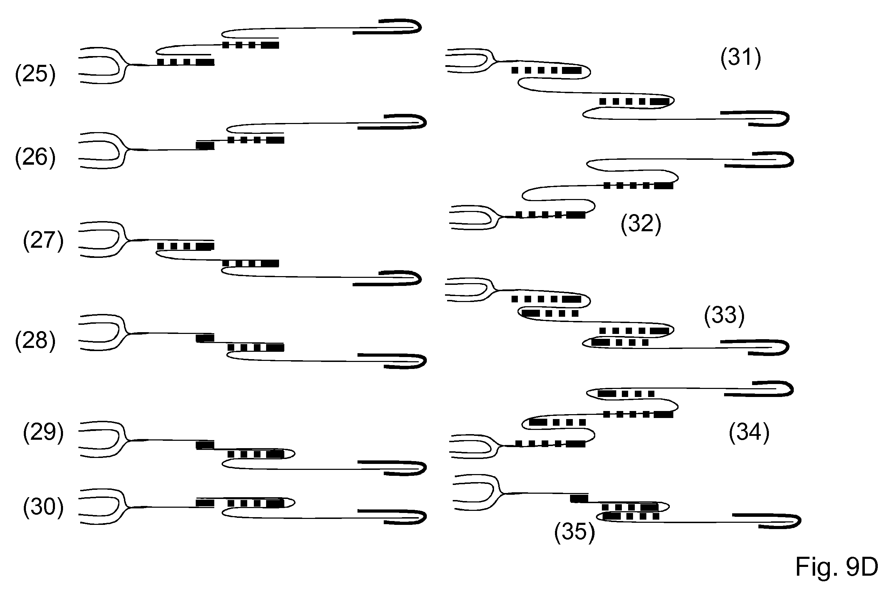

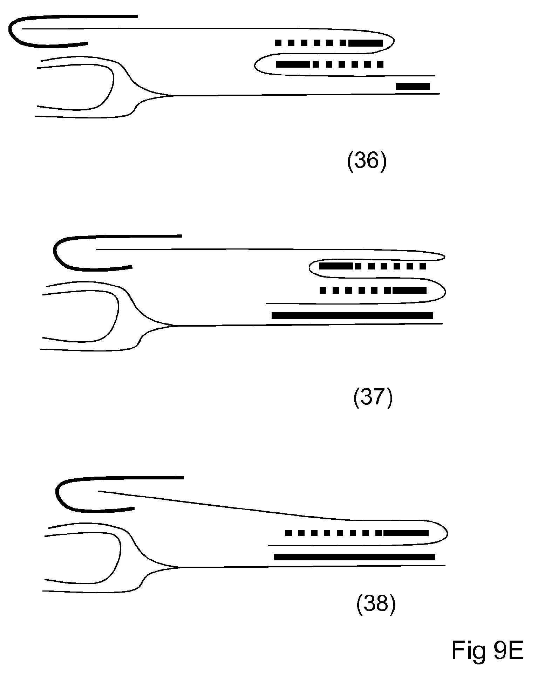

Optionally, the (front and / or rear) sidepanels may be composed of more than one web which partly overlay each other and are attached to each other as described above, and - non-limiting - depicted in Fig 9D and E.

Optionally and further enhancing the adapting to the body contours, the contouring attachment can be executed such as by shaped or contoured attachment, such as adhesive or heat bonded lines or regions. Upon donning and wearing the article, this creates an effect very similar to the one of darting or tucking in tailoring. This shaped attachment can be achieved such as by a curved attachment line, or by predetermined irregularly shaped attachment regions. Thus, when considering an article in its in-use configuration - i.e. when the centre piece to side panel connection is cross-directionally pulled out - the distance between the longitudinal centreline and the innermost demarcation line of the innermost of the contour attachment lines or regions is different in the hip regions from the corresponding distance in at least one of the waist or hoop regions. In the present context, the term "innermost" refers to the position relative to the longitudinal centre line of the article.

Optionally, the present invention can very suitably be used for designing articles, such as disposable absorbent articles, comprising a so called secondary topsheet for separating faeces from the skin of the wearer.

In yet a further option, the present invention allows designing of pants or pants-style structures comprising well fitting leg hoops.

The present invention relates to articles which may be closed pants or pants-style articles or to articles, which are open products, and which may be brought into a closed pants-style form upon donning. The present invention further relates to pre-forms of such articles, which may require addition of certain elements or performance of certain process steps to be functional as an article.

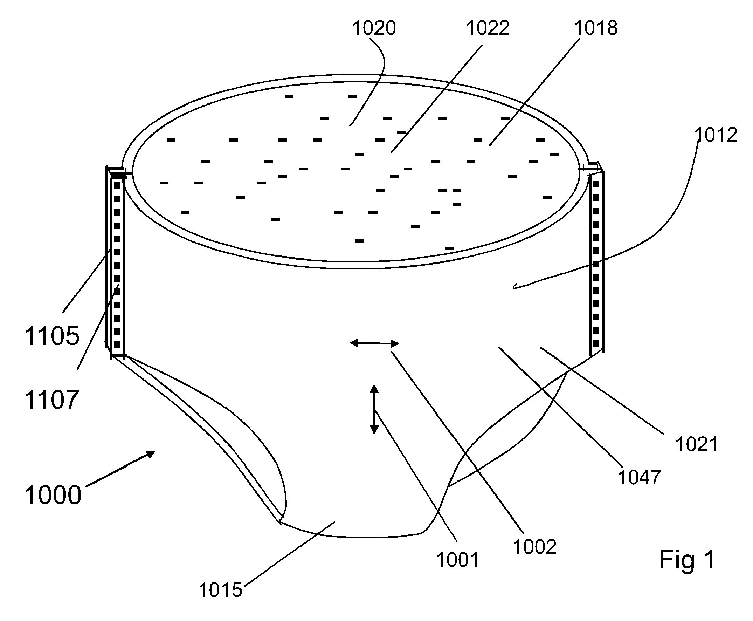

The general features of a pants-style article are depicted in Fig. 1, showing exemplarily for closed pants, such as an absorbent article, such as so called training pants, an article 1000 having a front waist region 1012 and a rear waist region 1018 connected by a crotch region 1015, thereby defining a longitudinal (x-) direction 1001 of the article. A width (y-) direction 1002 of the article corresponds to the right-left orientation on a wearer. Typically, the thickness (z-direction) of the articles is much smaller than the length and width, and extends perpendicularly thereto.

The articles according to the invention exhibit a wearer oriented, or inner surface 1020, in the context of disposable articles such as diapers also referred to as the topsheet side, and an opposite or outer surface 1021, in the context of disposable article also often referred to as backsheet side.

Such pants-style articles may be closed by side closure means, here shown as a butt-type side seam 1105 with connecting points 1107. In this butt-type seam the inner surfaces of the front and back regions are contacting each other and bonded to each other, such that outer margins of the webs form the distal end of the seam and extend outwardly away from the wearer during use.

Within the present context, the orientations relative to the article during its use are also applied to an article prior to the wearing. For most articles, the determination of longitudinal respective cross-directional orientation and of inner and outer surfaces can be straightforwardly deduced from the intended use by a skilled person. For certain articles, such as articles constructed of essentially one sheet of material, or designed symmetrically in the front and back, the orientation may be defined so as to provide the best comfort for the wearer, such as determined by outwardly lying butt seams.

Articles according to the present invention may have absorbent elements, as is the case for disposable absorbent articles such as baby or adult incontinence diapers, or for training pants and the like. Such articles may also be designed without integral absorbent elements, but may be combined with separate absorbent elements, such as when pants are used in combination with absorbent pads for the use in the context of adult incontinence or for feminine hygiene. Such articles may also be designed with no or relatively little absorbency, and may be used as disposable, or limited re-use underwear. Articles having no or only little absorbent capacity may be designed to withstand at least a limited amount of wash cycles without disintegrating.

In the case of pants or pants style structures these may be manufactured as completely closed pants - either permanently or releasably - or in an opened configuration, such as conventional baby or adult incontinence diapers. Such articles are closed to form a pants-style structure by the user or a caretaker upon donning of the article.

Pants-style articles according to the present invention comprise a centre strip and side panels. Both the centre strip and the side panels have major inner surfaces (i.e. predominantly oriented towards the wearer during use) and major opposite surfaces generally oriented away from the wearer. Within the present description, the term "major" surface refers to the orientation of a surface of a material or an article, which does not exclude, that certain portions of the material or surface are oriented differently, such as when a part of the material is folded away. No more than 50% of the material, typically less than about 25% and often less than about 10% of the total material should exhibit an orientation different from the major surface.

It is a particular design element of pants-style articles according to the present invention that due to the double pleating the side panels may be connected to the centre strip with their opposite surface oriented towards the opposite surface of the centre strip, e.g. backsheet side to backsheet side. This has particular advantages during the use of the article, as upon closing of the sides of the article either during manufacturing or by the user the lower section of the side panels cooperates with the crotch region of the centre strip to allow snug fit around the legs of the wearer.

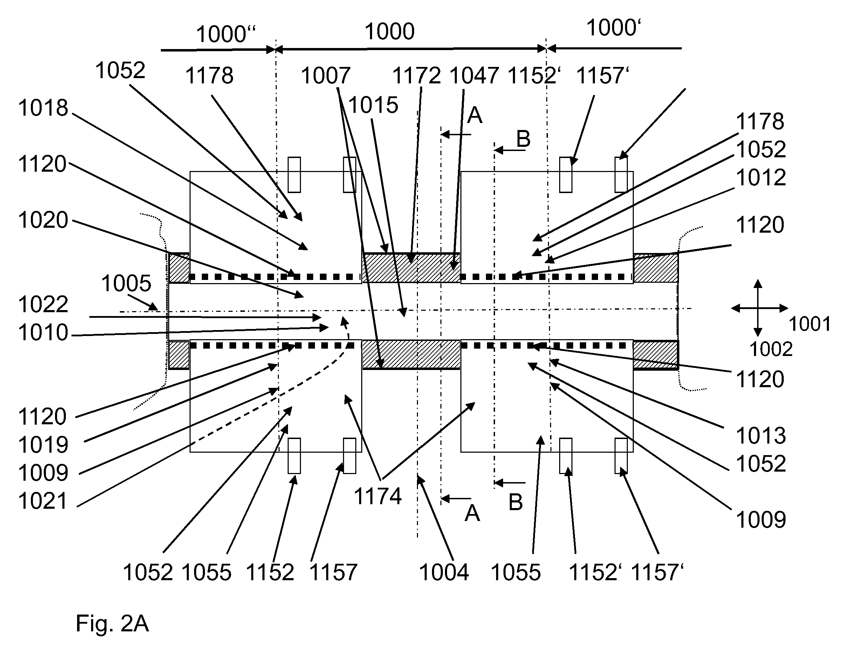

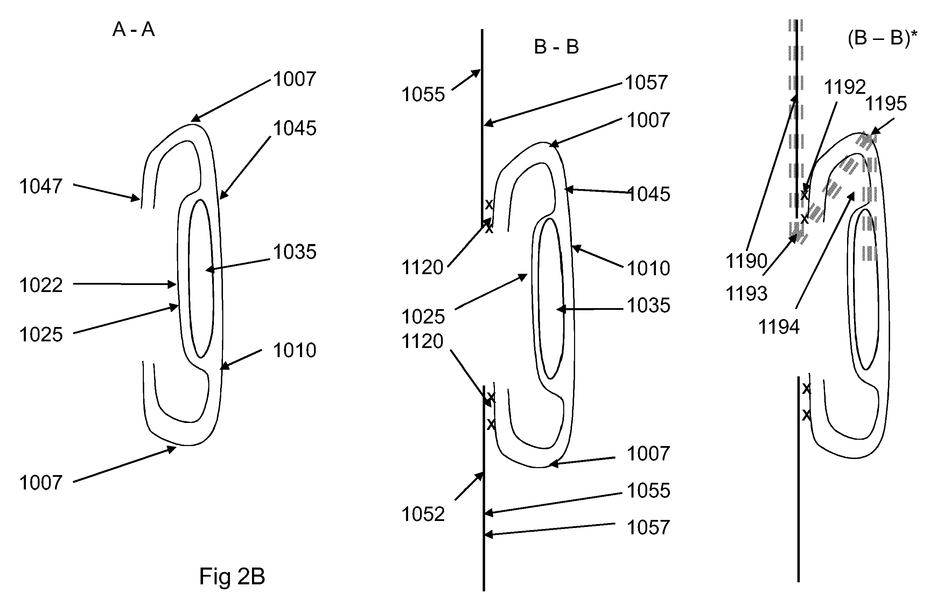

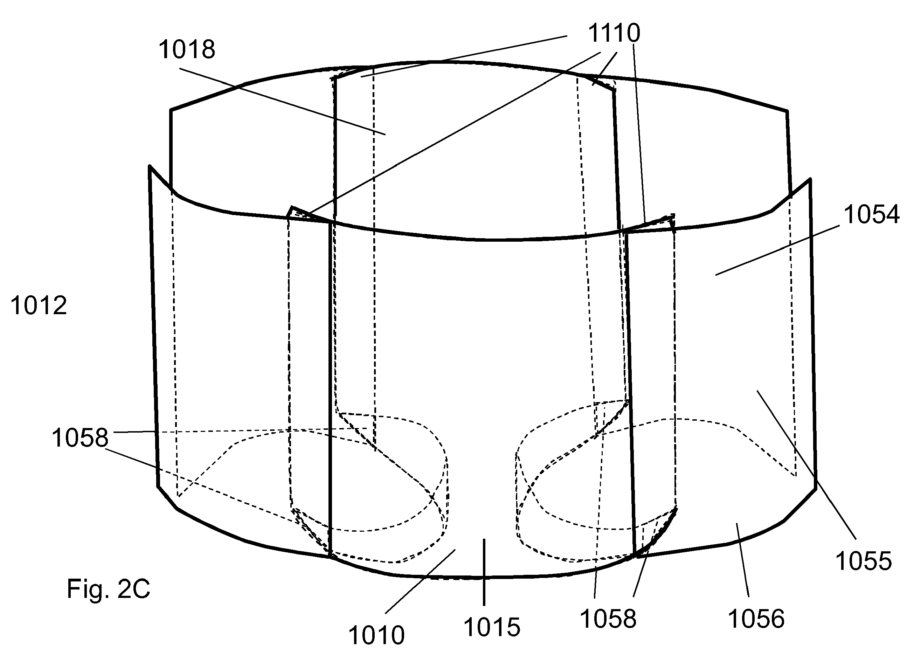

An exemplary design according to one embodiment of the present invention is shown in Fig. 2, showing a precursor of an article during a manufacturing step. It is symmetrical along its longitudinal centreline 1005. Fig. 2A exhibits a top view of the inner surface 1020 of a precursor 1000 according to the present invention (i.e. the general orientation towards a wearer). The precursor is shown during its manufacturing as a segment of a continuous web still connected at the segment demarcation lines 1009 to a leading segment 1000' and trailing segment 1000". The segment may correspond to a finished article just prior to its final cutting step. As shown, the article may be an open type diaper - here shown with upper (waist) closure tapes 1157 and lower (leg) closure tapes 1152 and with corresponding tapes 1157' and 1152' for a leading segment.

Along the longitudinal centreline 1005 of the article 1000, the precursor has a front 1012, rear 1018 region and a crotch region 1015 positioned there between. As also can be seen in the cross-sectional views in Fig. 2B, the precursor has a centre strip 1010, here shown as a composite of a topsheet 1025, a backsheet 1045 and a core 1035 enveloped there between. The elements of the centre strip may be suitably connected to each other, such as by gluing, as well known in the art.

The precursor as shown has four side panels 1055 connected to the centre strip extending in the front and back laterally outwardly of the centre strip, here shown longitudinally coextensive with the centre strip, thusly forming front 1013 and rear 1019 margin of the article, as shown in the figure coinciding with the segment demarcation line 1009. The precursor further comprises side panel hoop regions 1174 extending in the side panel regions from the cross-directionally extending crotch margin away from this margin and centre piece hoop regions 1172 essentially connecting these along the longitudinally extending side margins in the crotch region. Further, the precursor comprises hip regions 1178 essentially positioned between the hoop regions and the waist regions.

As can be seen in the cross-sectional views AA and BB, the centre strip is folded along longitudinal foldlines 1007. The width of this folded-over portion determines the size of the overlap area in which centre strip and side panels can be connected. As such it also influences the contour adapting effect of the side panel to centre strip connection, as being discussed in more detail herein below. For a baby size product, the overlap may be between 3 and 50mm, for an adult size product it may be between 3 and 100mm.

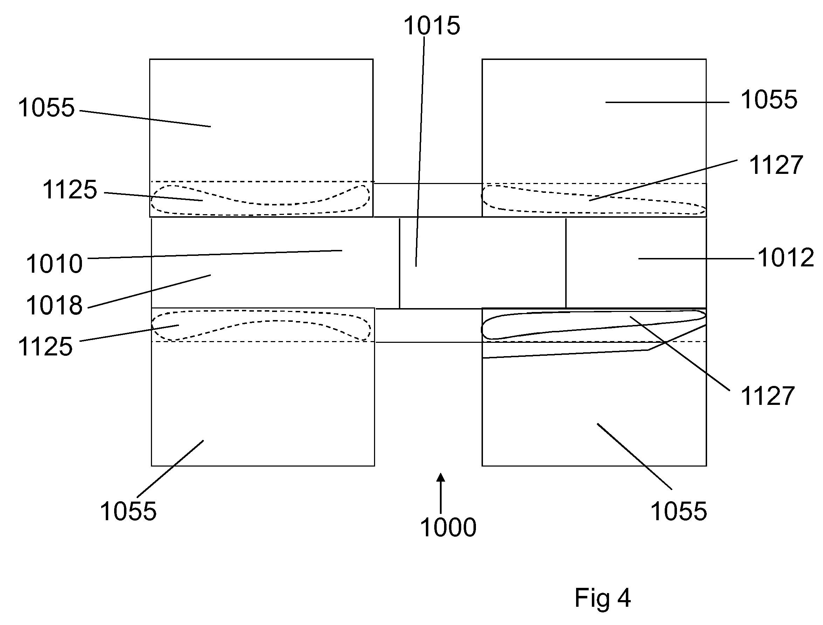

The side panels are connected to the centre strip via connecting regions, here shown by side panel attachment lines 1120 (shown as a dotted line in the top-view, as they are not visible therein, and as xx in the cross-sectional view). The connecting regions as indicated in Fig. 4 extend preferably between 0 and 10 cm inwardly of the longitudinally extending side margins. Preferably the inwardly oriented (i.e. towards the longitudinal centre line) perimeter of the connection region has a varying distance to the longitudinally extending side margins and/ or centre line.

The centre piece and the side panels are connected so as to form a double pleated (or Z-) fold. In the cross-sectional view (BB)*, which otherwise shows the same features as (BB), this is indicated for one side of the article by the thick dashed line 1190, with a first pleat 1192 and a second pleat 1194 with the respective first pleat tip 1193 and second pleat tip 1995. Attachment glue is positioned in the first pleat 1192. This embodiment shows the second pleat tip 1195 as a fold line of the centre piece material, whilst the first pleat tip 1193 is formed by a butt-type attachment of two materials (i.e. centre piece and side panel), looking as if the pleat tip of a continuous material would be clipped off.

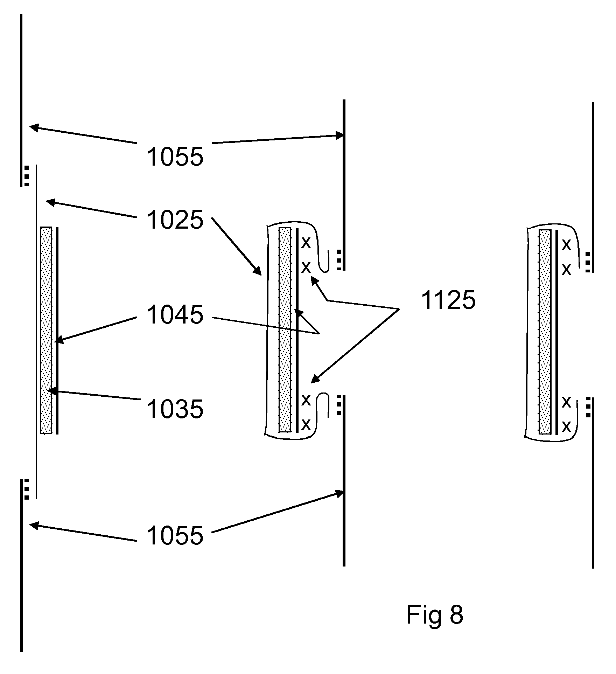

In this design, the side panels 1055 are oriented such that they exhibit a first major surface 1052 oriented towards the wearer and in the top view Fig. 2A shown facing upwardly. Similarly, the centre strip has the major wearer oriented surface of the topsheet 1022 facing upwardly in the cross-directional mid portion of the centre strip, whilst this surface is partly covered by the longitudinally folded side portions of the centre strip, where the opposite surface of the backsheet 1047 (hatched in the top view) faces upwardly. Further variants of this design are shown in e.g. Fig. 8 and Fig. 9A(9) and (10). Therein, a wider topsheet 1025 is folded around the longitudinal side edges of the centre strip, and the folded over regions are attached to the backsheet 1045 by means of a bond line or area essentially parallel to the longitudinal centreline of the article, the folded over parts of topsheet 1025 would be considered part of the backsheet. In a further embodiment, wherein a wider topsheet 1025 is folded around the longitudinal side edges of the centre strip, the folded over regions are attached to the backsheet 1045 by means of a shaped bond area 1125 as depicted in Fig. 4, and to the side panel by means of an essentially straight bond line or area, the folded over part of the topsheet is considered to be part of the side panel.

Thus, the connecting of the side panels to the centre strip is performed by attaching the majorly outwardly oriented surface of the side panel to the majorly outwardly oriented surface of the backsheet. It should be noted, that these connected portions belong to the majorly outwardly oriented surfaces, even though these may have a differing orientation during or after the connecting. Thus, the side panel and the centre strip are connected to each other in a butt-type seam 1110 (in Fig.2C). This is a particular advantage, as it allows a broader selection and/or easier bonding means or tools for the side panel and backsheet materials, respectively.

This has to be seen in contrast to "conventional" designs as well as to the "body conforming article designs", wherein the inwardly oriented surface of the side panel is connected to the outwardly oriented surface of the backsheet, or the outwardly oriented surface of the topsheet. If the side panel in conventional products is placed between top- and backsheet, its outwardly facing side is connected to the inwardly facing side of the backsheet, and its inwardly facing side is connected to the outwardly facing side of the topsheet. In contrast to the early pleated "centre glue" designs with an attachment glue in the crotch region, the present invention achieves different effects by affixing the pleats between centre piece and (unitary or separate) side panels outside of the crotch region.

Thus, at the latest upon donning of an article according to the present invention, the waist panels are positioned so as to encircle the waist of a wearer, and is further affixed with closure means, such as tapes, here shown as an upper tape system 1152 which may close the article around the waist, and a lower tape system 1157 for closing the article around the legs.

When pulling the article tight in the front and rear waist regions, such as upon donning, the double pleat fold of the centre strip and/or side panel will partly unfold, according to the contour attachment pattern between centre strip and side panel, thus allowing to use the desired width of the combined centre strip and side panels for encircling hips and waist.

In a particular embodiment, the side panels reach well into the crotch region of the article, i.e. below the crotch line on a standing wearer, such that the gap between the crotch portions 1056 of the side panel is relatively small. The optimal dimension of this gap depends on the overall size of the article, and in case of an article fitting babies, such as a baby diaper, in which an overall waist circumference may range from about 350 mm to 700 mm, or be about 450 mm, the gap may be from about 70 mm to about 180 mm, or even between about 100 mm and 140 mm. In case of an article fitting adults where the waist circumference may range from about 700 mm to about 2100 mm, or be in the range of about 1000 mm, the gap may be less than about 290 mm, such as in a range of about 200 mm to about 260 mm. Such a "long" side panel provides a particularly advantageous interaction with the crotch portion of the centre strip and the closure system as it will cooperatively with these elements provide a snug fit around the upper thigh. The leg closure systems as may be made by tapes or by the side seam will inevitably pull the inwardly folded area of the centre strip outwardly and against the inner leg, thus forming an essentially triangularly shaped folded over area 1058 at the lower edge of the folded over side panel attachment area, such that this triangle 1058 is positioned with its inner surface against the inner leg. As indicated in Fig. 2C this generates a snug fit around the leg, maintaining good contact between the leg and the centre strip side areas.

Thus, a product design according to the present invention allows to broaden the selection of side panel and backsheet materials and provides a simple, and well fitting design compared to conventional and to the recently published "body conforming" articles.

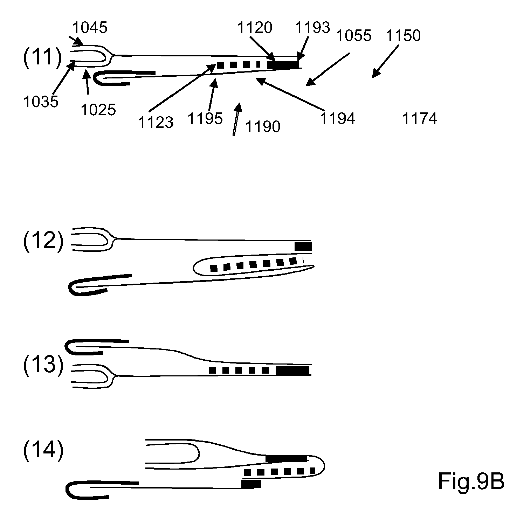

Instead of folding the centre strip and combining it with an unfolded side panel, the skilled reader will readily recognize for particular embodiments that the double pleated fold may be achieved by leaving the centre strip unfolded and combining with a side panel, where the side margins are folded over so as to allow attachment of the respective outer surfaces to each other, as shown in Fig 9 (36) to (38), without any limitations to these particular embodiments. Thus, comparing this to the design shown in Fig. 2B (BB)*, the pleat would look as if the other tip of the double pleat would be clipped off.

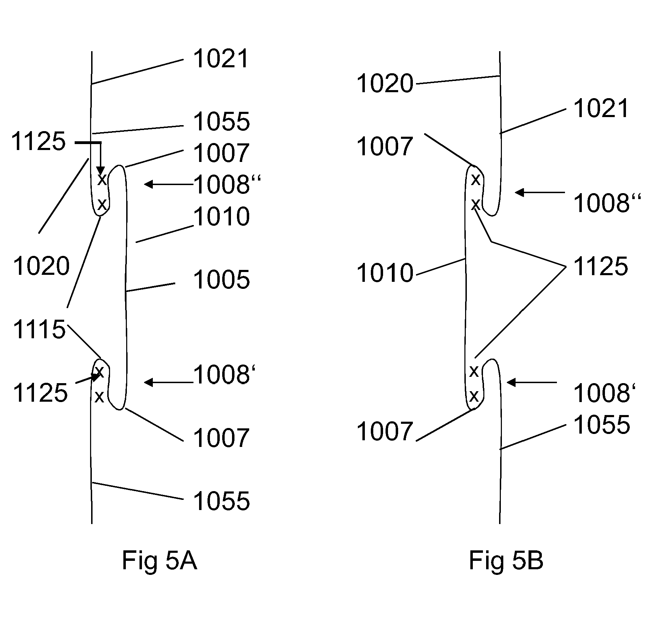

As shortly described in the above, the side panels may be essentially unitary with the centre strip, i.e. these may be made from the same web material without requiring particular connecting or at least comprise one material, which extends over both regions. In this case, the double pleated longitudinal folding may be executed in two variants, differing in the direction of the fold relative to the inner surface 1020 respectively outer surface 1021 of the article. The "upward Z-fold" can be seen in the lower part of Fig. 5A, which may correlate to the left part of the article during use. It will be mirrored symmetrically in the upper part in the figure, respectively right part in the article. The fold is such that - relative to the centre region around the longitudinal centre line 1005 of the article - the laterally outward regions are folded such that the major topsheet surfaces are upwardly folded onto each other. Conversely, for the "downward inverted-Z fold" (looking like a mirrored "Z" as can be seen in the lower part of Fig. 5B, the major backsheet surfaces are folded downwardly onto each other.

In this case of the unitary design, the general connecting of the centre piece and the side panels can be considered to be already given by this unitary material. In order to affix the pleat, the attachment may be achieved such as by an attachment glue 1120 (see Fig. 9 A(5) to (9), which may be integral with, identical with, or separate from the contour attachment.

In either of the options as shown in Fig. 5, the attachment of the pleats may be executed as lines but may preferably be executed as connecting regions 1125, thereby creating the bulging effect as described herein above in the context of non-unitary side panels. The butt seam connection as shown for the non-unitary designs is effectively replaced by a further longitudinal fold line 1115 in Fig 5. The connection between the side panels and the centre strip will form a "butt-type" seam (1110 as indicated in Fig. 2C) for a non-unitary side panel, and a kind of dart-type seam (as known from sewing) for the unitary execution. The two kinds of seam function essentially in the same way - and when pulling the article at its waist circumference, the unconnected parts of the folds will partly unfold along essentially straight or slightly curved lines formed by the side panel and the centre strip connected by the butt-seam or dart-seam. Thus, a butt-type seam looks like a particular execution of a dart-type seam, for which the outer end of the dart is cut open.

It is one particular benefit of the present invention to allow designing shaped articles, where a bulge adopts well to the body contours. A first execution of this embodiment relates to pants-style structures, such as described in general terms in the above. In order to adapt to the shape of the buttocks, the side panel connecting line (1120 in Fig. 2B) is modified so as to form a connecting region 1125 in Fig. 4 in the back region 1018 of the article. As shown, the shape of this connecting region is such that it is wider towards the rear and the front and narrower in the mid portion thereof. The front connecting region 1127 in the front region 1012 of the article may be straight, curvilinear, or a differently shaped connection area. Upon unfolding and donning, this particular connecting will create a bulge shape adapting very well to the buttocks and belly of a wearer.

Typically, for articles with non-unitary side panels the width (i.e. across the y-direction of the article) of the connecting regions 1125 and 1127 will be at least 1 mm, and may often be in the range between 5 mm and 60 mm. The rear connection region 1027 may e.g. be shaped such that it is wider in the waist region compared to the crotch or leg region, and even narrower in between. Thus, the distance between the longitudinal centreline and the contour attachment line respectively the innermost delimitation (i.e. closest to the longitudinal centreline) of the attachment region, which forms the first effective pleating, is different in the hip regions than compared to either or both of the waist or hoop regions. For articles with unitary side panels /centre piece design, the attachment line or region may actually be non-continuous between the waist and the hoop regions, i.e. there may be - and for certain designs preferably is - an interruption thereof, i.e. an attachment free region, such as in the hip region. During use, the pleat is in this region then essentially fully unfolded, whilst the pleats in the waist and/or hoop regions are still maintained, the shape contouring in the hip region is achieved with utmost material use efficiency. When present, the CD-width of the attachment lines or regions may often be ranging up to 60 mm.

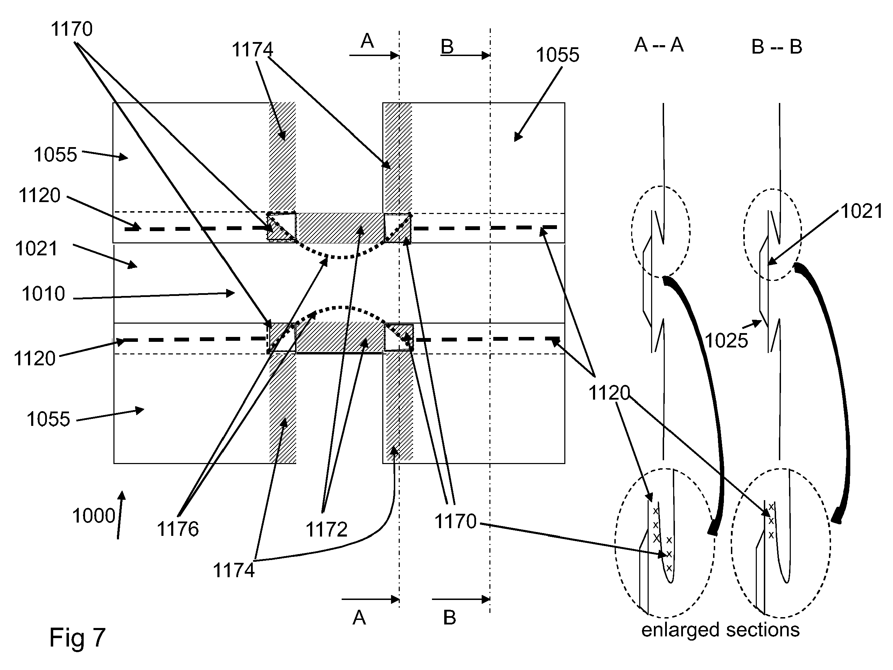

A particular embodiment of the present invention, which is further explained by referring to Fig. 7, relates to the design of an article 1000, wherein the leg encircling hoop is formed by parts of the centre piece and parts of the side panel material. To this end, the centre piece 1010 may be laid out flat before the folded side panel material 1055 is added. However, in addition to the side panel glue 1120, which connects the side panel to the centre piece and which may be applied in any of the options as described in the above, the side panel is tacked to itself in the side panel tack regions 1170. In the top view of Fig. 7, the centre piece strip 1010 is shown with its outer surface 1021 facing towards the viewer and the user oriented surface 1025 facing away. As described in the above, four pieces of side panels 1055 are folded and connected to the centre piece by side panel glue, here shown as glue lines 1120 along the full length of the side panel, although glue regions maybe used, too. Further, four side panel tack regions 1170 are shown, here shown in a triangular shape, whilst other shapes or even a connecting line such as a glue bead or ultrasonic welding line would be possible. The panel tack regions are preferably delimited by a line corresponding to a "curvilinear fold line", indicated by the dotted lines 1176 in Fig 7 (which develops once the article adopts to a three-dimensional shape, such as described in more detail in WO06/102974), and further by the side panel foldline, and a hypothetical line connecting the lateral outward points of the centre piece where the "curvilinear fold lines" terminate. If the connecting extends significantly beyond this region or lines, it may negatively impact the "improved fit function" of the SP. In these tack regions, the folded side panel material is securely connected to itself, which may be achieved by any conventional tools, such as glue application, welding such as by ultrasonic bonding and the like. A narrow, in use overfolded piece of the folded side panel (shown as a white triangle adjacent to bond area 1170) is attached to the centre piece and follows the inner hoop, so it should not be attached to the other surface of the SP. The function of the tack regions 1170 is to allow the forming of leg encircling hoop made up of the laterally outward portions of the centre piece in the crotch region 1172 and the respective portions of the side panels 1174 as indicated hatched in Fig. 7, which will - upon donning and closing - form a right and left hoop fully encircling the leg with an essentially closed force connection line (not shown).

A further embodiment of the present invention relates to a "detached topsheet", i.e. to articles wherein "topsheet refers to the uppermost layer, i.e. the layer which is positioned towards the wearer and which is intended to be in contact with the skin of the wearer. The term "detached" refers to the fact, that this layer is only partly connected to the base, with at least a portion in the crotch region along the longitudinal centre line being unattached to the base, but is rather connected to the overfolded side margins of the base. Thus, during use, a pick up connection, such as a glue line or dot, lifts the detached topsheet away from the base, thusly creating space for receiving bodily exudates more efficiently.

A first execution of this detached topsheet relates to a so called "BM trap" or "secondary topsheet". Such features are generally known to a skilled person and are described such as in US5037416, US5462541, or US6152907 or copending patent application GB1007486 (unpublished).

Such designs refer to articles, which during use provide a separation of faeces from the skin of the wearer, in particular in the genital region. The present design achieves this separation by including a secondary topsheet in the crotch region, overlaying (towards the wearer) the primary topsheet. This secondary topsheet may be positioned forward and/or backwards of the anal opening during use. It is affixed to the primary topsheet or the base along its longitudinal edges and optionally its front edge and/or rearward edge.

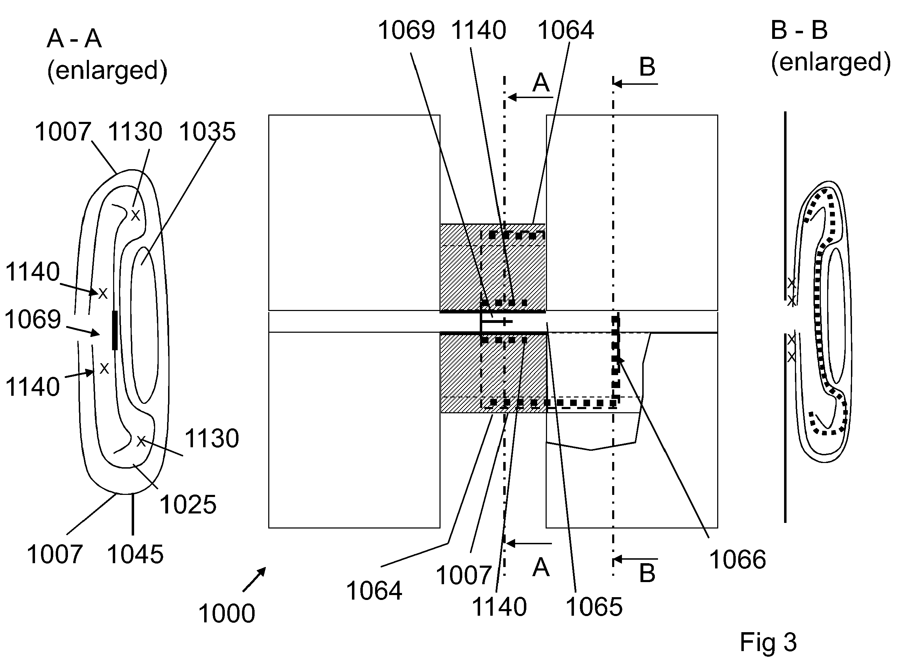

Fig 3 shows an article 1000 corresponding to the one of Fig. 2 (with respective features being not referenced in Fig. 3), but further comprising a secondary topsheet 1065 which is positioned on top of the primary topsheet 1025 cross-directionally inwardly of the longitudinal fold lines of the centre strip 1007, such that the folded over portions of the centre strip overlay the secondary topsheet (henceforth the perimeter of the secondary topsheet shown as dashed line in the top view). In this design a secondary topsheet fixation glue 1130 is positioned along the longitudinal margins 1064 and the forward margin 1066.

In this design a secondary topsheet pickup glue 1140 is positioned on top of the secondary topsheet. Optionally a secondary topsheet slit or opening 1069 may extend along the longitudinal centre line from the rearward edge forwardly towards the genitals, but preferably not into the area of the genitals. Alternatively, the secondary topsheet may also be fixed along its rearward edge against the primary topsheet. In this case it needs to be equipped with a slit or opening in the anal area.

Upon donning, the snug fit around the legs will appear, but at the same time the secondary topsheet will be lifted away from the primary topsheet by action of secondary topsheet pick up glue 1140, and the side areas close to the slit or opening in the secondary topsheet will be held against the legs of the wearer by the lower side panel areas transferring forces into the inner leg area of the centre strip side areas, thusly creating the aforementioned "BM trap", which can separate the faeces from the skin and genitals in the mid and front region of the article. The fixation of the opening or slit side margins to the leg surrounding centre strip side areas effectively causes the product width to self-adjust between the legs of the wearer.

In yet a further embodiment the articles comprise a donning aid, which may be a strip of web material, such as described for a secondary topsheet, and which is attached analogously thereto.

A further execution of the "detached topsheet" relates to a unitary topsheet, which is attached to the base in one part of the article, i.e. either the front or the back region longitudinally towards a discontinuity in the crotch region. The discontinuity is preferably executed as a cross-directionally extending cut or slit in the topsheet, but may also be an opening therein. On the opposite side of the discontinuity, i.e. towards the rear or front respectively, the topsheet is not connected to the base, but lifted by the pick up connection. Thereby, an opening is formed during use. If the forward part of the discontinuity is lifted during use, the BM trap, as described in the above may be formed. If the rearward part of the discontinuity is lifted, a urine receiving opening may be formed, particularly adapted to receive urine for a person in a supine position. A skilled person will readily realize, that the base needs to satisfy certain requirements with regard to having a surface adapted to be potentially in contact with the wearer, but also with regard to retaining ingredients such as fluff or superabsorbent materials therein. Also, the liquid handling properties of the layers may need certain adjustments such as with regard to liquid permeability - or lack thereof.

The articles according to the present invention may comprise absorbent elements, whereby the particular design allows particular executions as separate elements may be incorporated. Such absorbent elements are positioned between the outermost layer, often referred to as the backsheet, and the layer, which is positioned towards the wearer, often referred to as the topsheet. A first absorbent element is attached to the outermost layer at least in the crotch region, whilst a second element is attached to the innermost layer at least in the crotch region such that in the in-use configuration the second element is lifted away at least in a portion along the longitudinal centre line. Thus, as described for the detached topsheet, an opening may be formed during use such as for receiving faeces, if the secondary core is terminating in the perineal region and extends forwardly. Similarly, the secondary core may terminate in the perineal region during use but extend rearwardly, such that it can readily receive urine loading e.g. in a back-lying position of a wearer. In such variants, the secondary core may also function like a stiffening element as described herein above.

When considering the projection of the first and second absorbent elements onto the outermost layer, they may overlay, overlap in the crotch region or be spaced apart, though a gap in this projection should not be excessive.

Generally, there are no particular requirements for the absorbent elements. The first and the second absorbent element may be of the same type of material, and may - for example - be off the same roll of absorbent material.

In a particular execution the first and a second absorbent element are positioned onto a carrier web, such as a nonwoven web, which may be suitable as topsheet. The first and the second element may have be cut and spaced off a single roll of absorbent material. Alternatively, they may come from two different rolls, and may have different composition and/or dimensions. In a further alternative, one or both of the elements may have be formed "on-line", such as by a conventional core forming apparatus. The two elements may be positioned onto one web carrier or two web supports, which may move at differential speed, such that the leading edge of the slightly faster moving trailing element may have overlapped the trailing edge of the slightly slower leading element, such that the two elements are delivered to the other elements of the articles in an overlapping (or shingled) positioning. The carrier web between the elements can be folded in the longitudinal direction in a Z-folded manner. If the two elements are placed in the "detached topsheet" design as described in the above, and one of the elements is connected to the base in the crotch region whilst the other one is lifted, the carrier may form the opening and may be adapted to allow passage of liquids, like urine of faeces to penetrate through, such as by longitudinally extending slits between the elements. The cross-directionally extending edges of the elements may be straight in the cross-direction, but alternatively, they may have varying shapes, such as rounded, or angled, or chevron like. A skilled person will readily realize that also the trailing edge of the leading element may overlie the leading edge of the trailing element. A further particular embodiment of the design has side panels, which are connected by a so called "perfn pop" feature. This refers to a connection between a first and a second side panel, which can withstand the processing / manufacturing steps, but which can be readily separated by a user e.g. upon donning. Alternatively, this connection may already be separated at a final process step in the manufacturing. A "perfn pop" connection as such is well known for a skilled person. Typically, it is produced e.g. by treating a material such as by an interrupted separation line. Whilst some product options produced by such perfn pop processes may not show any difference in use as compared to products made from separate side panels, this design offers more process flexibility, particularly as far as side panel design options are concerned.

In any of the options for the side panels, these may be made of a single material, or a composite material. Such a material may have varying properties, such that strength or elasticity is higher e.g. in the waist region than in the crotch or leg region. Such a composite may be a multi-layer design, such as elastic strands being affixed between nonwoven materials. Such a composite may also be composed of materials having differing properties in a side-by-side arrangement, optionally connected to each other by a butt type, or an overlapping connection..

It should be noted, that they are variants of the present design principle, which are considered to be covered by the present invention.

Instead of the two tape system shown in the above, the article may comprise a multiplicity of tapes, or a single tape system. Optionally, two tapes may be connected at their distal ends, whilst the opposite ends are connected to the waist and the leg regions respectively.

Further, the article may comprise leg elastics extending longitudinally, which may be sandwiched in a conventional way between topsheet and backsheet adjacent to the longitudinal margins. Optionally, the elastics are folded into foldlines along the lateral margins of the secondary topsheet, or sandwiched between the secondary topsheet and the wearer facing surface of the base.

Further, the topsheet and backsheet materials may have differing widths, and one material may be folded along a further longitudinal folding line over the longitudinal edge of the other material.

Optionally, the topsheets may comprise a skin care composition, such as a lotion as well known in the art.

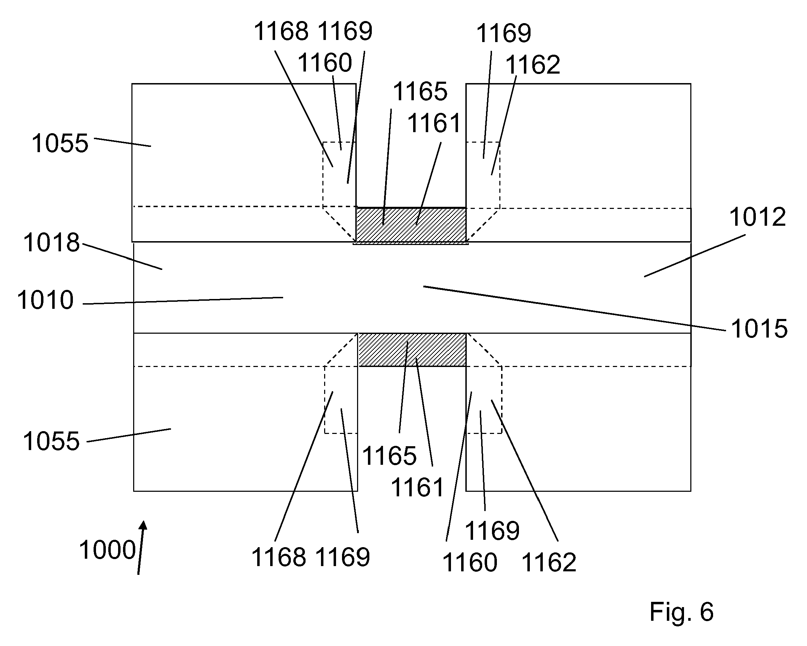

In yet a further embodiment, pants or pants-style articles may comprise leg hoops, such as described in general terms in WO06/102974. Fig. 6 shows an article corresponding to one as shown in Fig.2 (and hence duplicate numerals are partly omitted), additionally showing leg hoops 1160, which are folded over in their crotch region 1165 positioned in the crotch region 1015 of the article, such that a first leg hoop surface 1161 (hatched) is visible in Fig. 6, but are not folded over in the outwardly extending front 1162 and rear 1168 extensions, where the opposite leg hoop surface 1169 can be seen in Fig. 6.

In a second aspect, the present invention relates to the manufacturing of absorbent articles, which may be well employed for producing certain articles described above especially at high production speed. It is a particular benefit of the invention that for many of the embodiments shown, all components forming the precursor / article can be stacked up such that they do not exceed the limits of the centre piece. This allows respective machines to generate an output of 600 to 2400 pieces per minute, at web speeds of about 300 m/minute, or even more than 1000 to 4000 articles per minute, corresponding to manufacturing web speeds of more than 500 m/minute, for articles with a length of around 500mm.

It is well known in the art, that articles may be produced such that the orientation during production is aligned with the longitudinal direction of the article during use ("MD process"). Alternatively, in the so-called "CD process" the machine direction during production is perpendicular to the longitudinal orientation of the article during use.

Whilst the present invention is applicable to both a MD- and a CD- process, it should be noted, that for the purpose of the present explanation, the terms "longitudinal" (or x-direction), cross-directional (or y-direction), "inner" surface respectively direction, "opposite" surface respectively direction are defined in relation to a ready to use article, as described in the above.

The process of manufacturing comprises the step (a) of providing one or more essentially endless webs, which are to be converted into the articles. A first web may comprise an essentially endless series of segments such as pre-forms of articles, still being connected to each other. These pre-forms may essentially have all required elements or components to form the articles, or some components may be added in course of the described process or thereafter. Thus the first web may comprise one or more centre strips. One or more second web(s) may be provided from which the side panels can be formed. Each of the pre-forms has a front, a rear, and a mid region corresponding to the orientation during use.

In the MD process variant, the front regions of the article may correspond to a leading or trailing portion of the web segments. For simplicity, the present discussion assumes that a front region corresponds to a leading portion of the segments of the web.

In the CD process variant, the front regions of the article may correspond the a left or right portion of a segment of the web, and the leading and trailing portions of the segments of the web correspond to left or right portions in the product. In case of a dual-lane setup, a centre piece strip may be twice as wide (in the MD variant) or twice as long (in the CD variant) as the respective centre piece in the finished article.

For an exemplary description in the context of disposable absorbent articles, such as diapers, the first web may be formed of a backsheet, a topsheet, and absorbent cores there between. Thus, the web exhibits an "inner surface", i.e. a surface which, upon finishing of the process, will be oriented towards the wearer, typically represented by the topsheet. The first web will also exhibit a major "outer surface" typically represented by a backsheet and facing away from a wearer during use. Correspondingly, if a second web for forming the side panel is provided, it will also exhibit a major inner and a major outer surface

In such a web handling and treatment process, the web is typically supported by support means, such as may be support plates, transport belts or rolls and the like, all well known to a skilled person. For the purpose of the present discussion, these support means have a top surface over which the web is transported. Whilst for certain process steps the web may be located between support means (such as a pair of rollers or transport belts), the skilled engineer will readily recognize an "upper surface" of the web transport or treatment equipment, over which the web is transported. The web may be oriented towards this "upper surface" of the transport or treatment equipment such that either its "inner" or "outer" surface are in contact with or facing towards this upper surface.

In case the finished article contains leg hoops, and in case of making the product by stacking on an MD line, leg hoop strips being as wide as the leg hoop in the finished article (in case of a single lane converter), and in addition, one or more leg hoop strips being as wide or twice as wide as the leg hoop in the finished product (in case of a multi-lane converter), are laid with their major outer surface onto the treatment equipment.

The attachment may be created by contoured glued lines or by a continuous glue area, or other attachment connecting means like ultrasonic bonding, heat bonding, and the like. In a next step, sidepanel strips are laid down with their major inner surface facing the treatment equipment. Their width is corresponds to the sidepanel width in the finished article as described for the leg hoops.

In a next step, at least one centre strip web is laid down with its major outer surface onto the stack of leg hoops and sidepanels, and the treatment unit. In case of single-lane MD converting, the width of the centre strip equals the width of the centre piece in the finished product. In case of multi-lane converting with x converting lanes, the width of the centre strip is either x-times as wide as the width of the centre piece in the finished product, or x centre strip webs are supplied, each web being as wide as the centre piece in the finished article, or a combination of narrow and wide webs is laid down.

Subsequently, the components forming the article stack are connected, preferably by means of heat and/or pressure bonding such as ultrasonic bonding, or by glue bonding, provided glues were applied the respective webs before they were covered by an overlying stack component.

In a particular embodiment the attachment region may be formed by a bonding roll, such as may exert increased pressure and/or temperature against the treatment unit. The bonding roll may have a bonding pattern corresponding to the connection regions, or the pattern may be part of the treatment unit. The bonding rolls may have their rotational axes parallel to the axis of the treatment unit.

Again, it should be noted, that the terms "longitudinal" etc. refer to the orientation of an article during use, and may be parallel or cross-directionally oriented towards the machine direction of the process and equipment.

In addition, the process according to the present invention may comprise additional, optional process steps:
Thus, a secondary topsheet may be applied to the web. To this end, a further web material 1065 is provided and positioned by conventional tools, such as so called cut-and-slip, and positioned on the upper (topsheet) side. This secondary topsheet is positioned in a region corresponding to the mid region of the article in use, but optionally offset from a cross-directional centre line 1004 and essentially centred cross-directionally. This secondary topsheet may be combined with the primary topsheet or the base - in case the topsheet is equipped to take on the functionality of a secondary topsheet - by conventional means, such as gluing or heat/pressure bonding, along its front margin and along at least parts of its longitudinal side margins, and optionally at its rear margin.

Optionally, for making a closed pants-style article, the front and rear side panels may be connected to each other by conventional means, such as adhesively gluing, or (e.g. ultrasonic) welding or other means as well known in the art.

Optionally, adhesive tapes may be applied to the sidepanel strips, before these are cut from the sidepanel strip web, and laid down as discrete pieces onto the treatment unit, either a single tape on each side, or a dual tape system with two tapes on each side. The tapes may be conventional adhesive tapes or so called mechanical fastening systems, both well known in the art.

Further optional process steps may be employed to apply elastic components, such as at least in the crotch area, if not already applied to the web before the presently described process.

The topsheet and backsheet materials may have differing widths, and one material may be folded along a longitudinal folding line Alternatively, sidepanel webs may be composed of webs stacked upon each other e.g. such that their major outer surfaces are in contact, and one of the two lateral edges of the first sidepanel web is aligned with one of the two lateral edges of the second sidepanel web.. The webs are bonded along this very side, and subsequently cut to form sidepanel strips which are laid down onto the treatment unit.

It will also be apparent to a skilled person that the side panels do not need to be applied in rectangular form.

Optionally, a skin friendly substance such as a lotion may be applied to the wearer oriented surface of the topsheet(s). The resulting composite may also further be folded along its cross-directional centreline 1004, or along other lines, to make it fit a package.

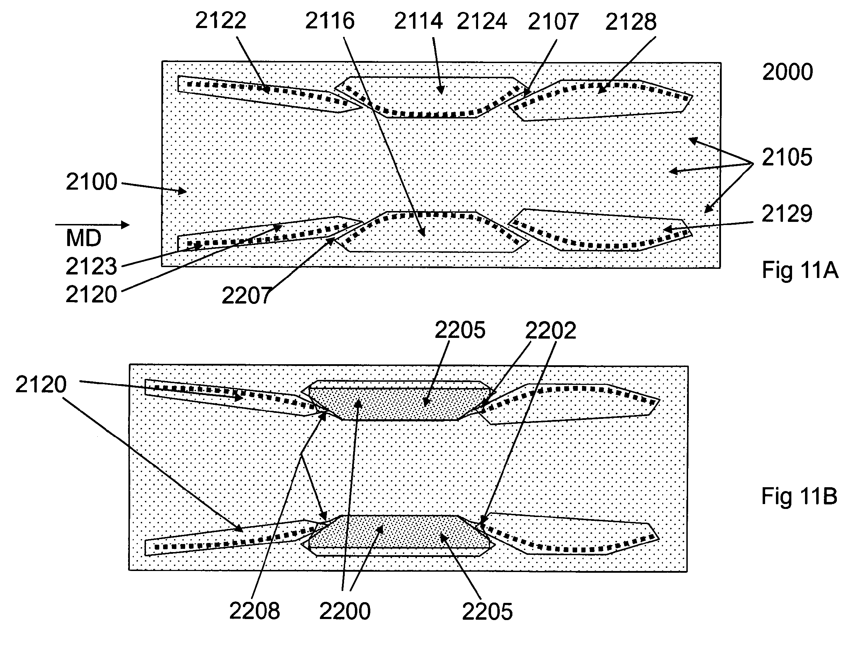

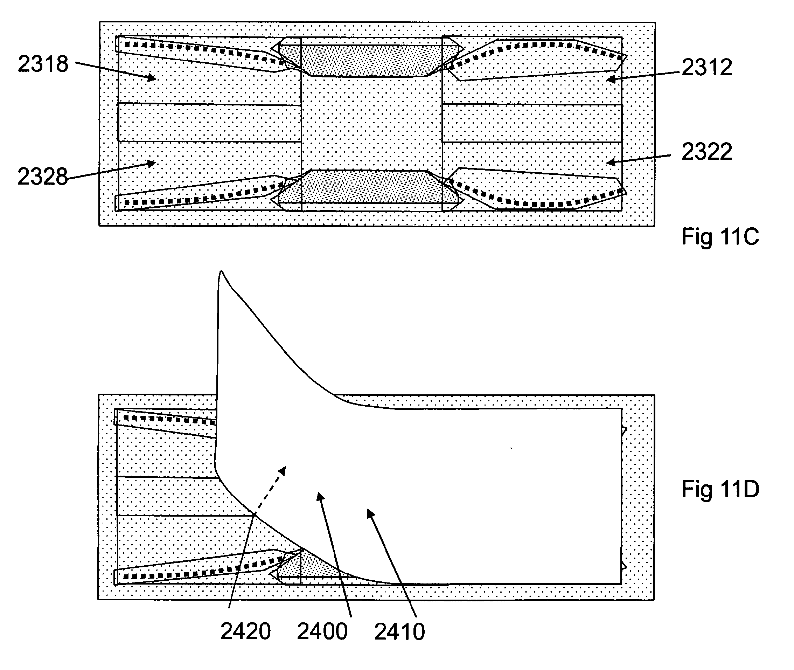

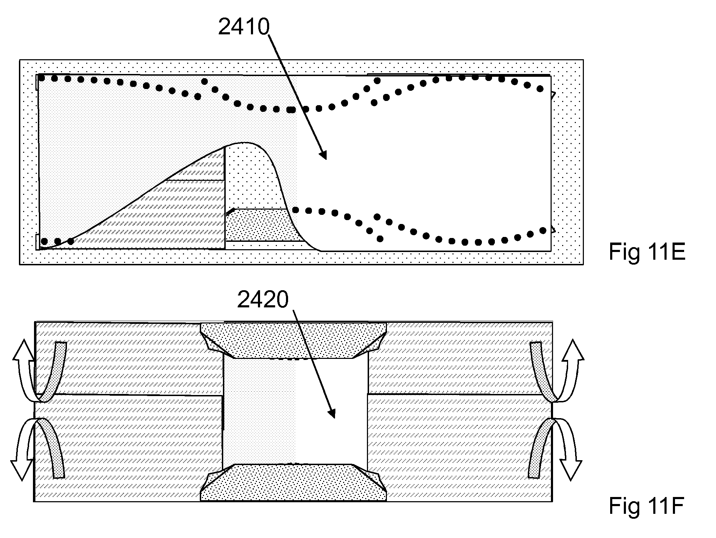

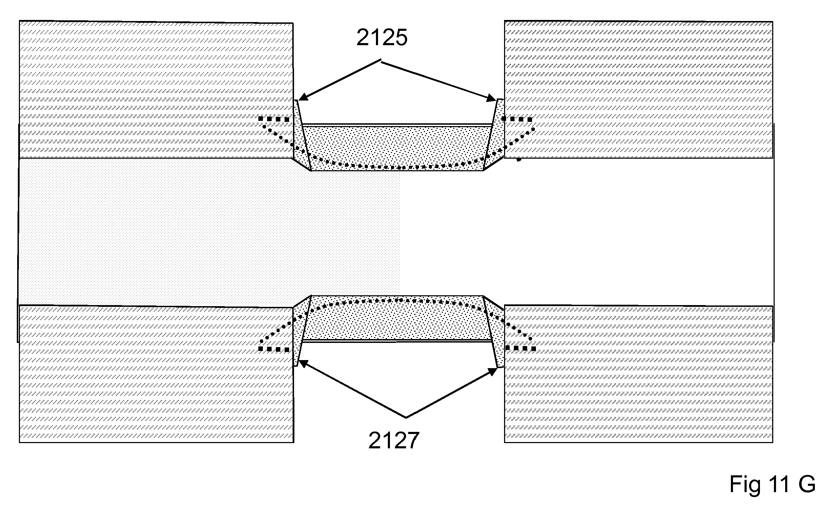

An embodiment of the manufacturing process according to the present invention relates to the making of articles comprising leg hoop as described in the above in the context of Fig 6 (which shows the article in a ready to use condition) in an MD-manufacturing process. Such an article can suitably be made by providing a manufacturing surface 2100 (refer to Fig. 11 A to G), such as of a turret, of a manufacturing belt or the like. The surface may be provided with web fixation means, here exemplarily indicated by vacuum holes 2110. These web fixation means may be separated into separately operatable or activatable regions, here shown for the first (2114) and second (2116) hoop region. The surface further comprises bonding means members, here exemplarily shown as anvils 2120 for ultrasonic bonding horns or sonotrodes (not shown). These anvils have forms corresponding to the respective connecting regions for the centre piece with the side panels, here shown for the first rear 2128 second rear 2129, first front 2122 and second front 2123 side panel. Further anvils 2124 and 2126 are in a form corresponding to hoop connecting regions. In a particular embodiment, these anvils are executed as metallic spirals, which may be positioned in grooves with an appropriate depth, such that the sonotrodes interact with the individual turns of the spiral and provide a particular bonding pattern, as described in more detail in co-filed GB application (application number yet unknown, attorney docket number BCT0701). As indicated in Fig. 11B, each one strip of hoop forming material 2200 is positioned over the hoop fixation region 2114 and 2116 such the mid region 2205 thereof is affixed thereto such as by vacuum suction. The front (2202) and rear (2208) parts of the hoop forming material are positioned between the hoop fixation region and the connecting regions into hoop gaps 2107, such as by vacuum suction, such that they extend through the plane of the manufacturing surface 2100 (here shown by the loose ends 2202and 2208, respectively). As the next step, indicated in Fig 11C, the side panels 2300 are positioned onto the surface, here shown with first 2312. 2318 and second 2322, 2328 front and rear side panels respectively, and are overlaid with the centre piece web 2400 which is (preferably) still a continuous web facing the side panels (and hence the support surface) with a second surface, in Fig 11D shown as a backsheet surface 2420. Thereafter, see Fig. 11E, the connecting is made along all connecting lines, such that the side panels and the hoop forming material are connected to the centre piece, still leaving the loose front and rear parts 2205 and 2208 of the latter unconnected in the hoop gaps. In order to further connect these loose parts to the side panels, the connected centre piece, side panels and hoop materials are turned, such as might be readily achieved by transferring the continuous centre piece to a further web support (not shown) such that now the second surface faces outwardly, now shown in Fig. 11F backsheet surface 2420 facing towards the viewer. The side panels are now taken up by a side panel turning means (not shown), such as gravity, or a guide rail or plough positioned / inserted between the side panels and the centre piece for folding the side panels laterally outwardly, as indicated in Fig. 11G. This folding will inadvertently also fold the loose ends of the hoop forming material outwardly such that they can be connected to the side panels outwardly of the centre piece. by a further connecting means, such as indicated hoop ends bonding 2125 and 2127.

It is a particular benefit of the method described above that all materials composing the article can be stacked up within the longitudinal and cross margins of the centre piece. Consequentially it is possible to convert several of such article stacks side by side at the same time, sometimes referred to as "multi lane converting". This setup has particular benefits in the context of an MD manufacturing process without being limited thereto - as it enables producers to increase the output of a machine by a factor of two, three, or even four, without increasing the overall web speed on the line,. Alternatively, multi lane converting allows lower web speeds - compared to single lane converters - without reducing the overall output of a machine.

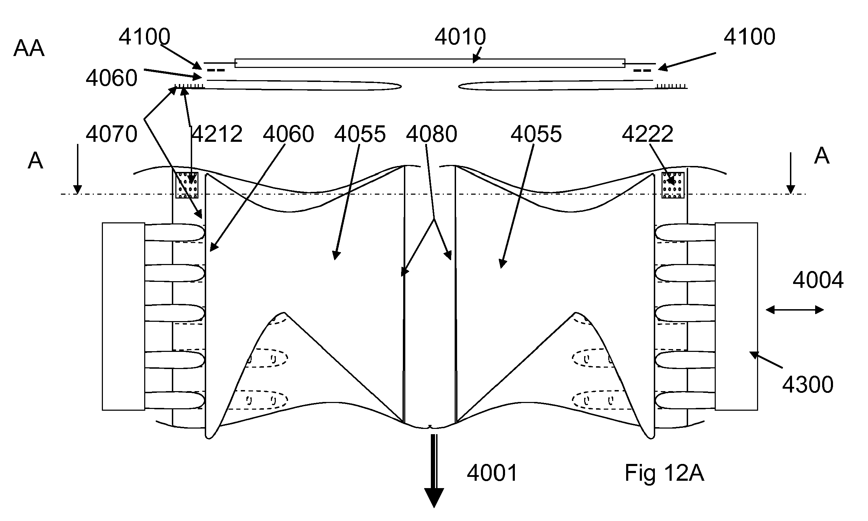

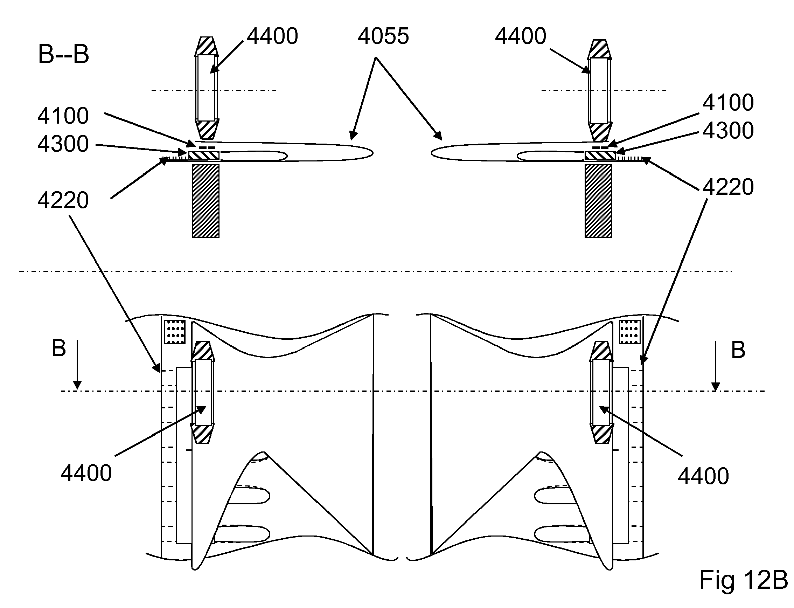

Yet a further variant of the manufacturing process relates to the case of making closed pants style articles, or so-called pre-closed diapers, in a single-lane MD process. In the cross-sectional view AA of Fig. 12A, the centre piece 4010 comprises an absorbent core and a topsheet and backsheet, extending laterally outwardly of the core. Side panels 4055 are shown in a folded configuration, with a first longitudinal side margin 4060 positioned in registry with the centre piece to allow connecting across the side panel to centre piece connecting region 4100. The figure shows a composite web comprising centre piece and side panels positioned ready to be combined but not yet connected. Corresponding regions can be joined on each side of the article, in Fig. 12B shown for regions 4220. This joining can be executed as a permanent bond, such as be melt fusion. Alternatively or additionally it may also be achieved by openable and recloseable connections, here indicated by hook patches 4212 and 4222 allowing to manufacture pant style closed articles which can be readily opened and reclosed by a user or a caretaker.

As the web moves along machine direction 4001, the configuration as shown in Fig 12A can be readily achieved by the principles discussed hereinabove. However, as on each side the first of the longitudinal side margins of the side panels extends laterally outwardly of the longitudinal side margin of the centre piece (which is in registry with the second longitudinal side margin of the respective side panel), the connecting of the side panel to the centre piece in the connecting regions 4100 carries at least the risk of inadvertently connecting all three layers (i.e. also the two layers f the side panel material) together. In case of using glue bonding, the risk is the so called glued bleed through from the connecting region through the side panel material. In the - otherwise preferred - case of melt fusion bonding, the three layers will almost unavoidably stick together.

In order to still be able to execute such processes, a separation means 4300 is introduced between the layers of the side panel material. A indicated in Fig 12A, the separation means 4300 can comprise finger shaped plates 4350, which are cross-directionally moveable, indicated by arrow 4004. In Fig. 12A, they are shown laterally outwardly of the first longitudinally extending side margin 4060 of the side panel. As shown in the cross-sectional view 12B, the fingers are moved laterally inwardly and are now in registry with the side panel to centre piece connecting region 4100. A connecting tool, here shown exemplarily as the sonotrode 4400 of an ultrasonic welding equipment, may interact with the finger plates 4350, which now act as anvils for the connecting tool, and the connecting can be accomplished without connecting through all layers of the side panel material.

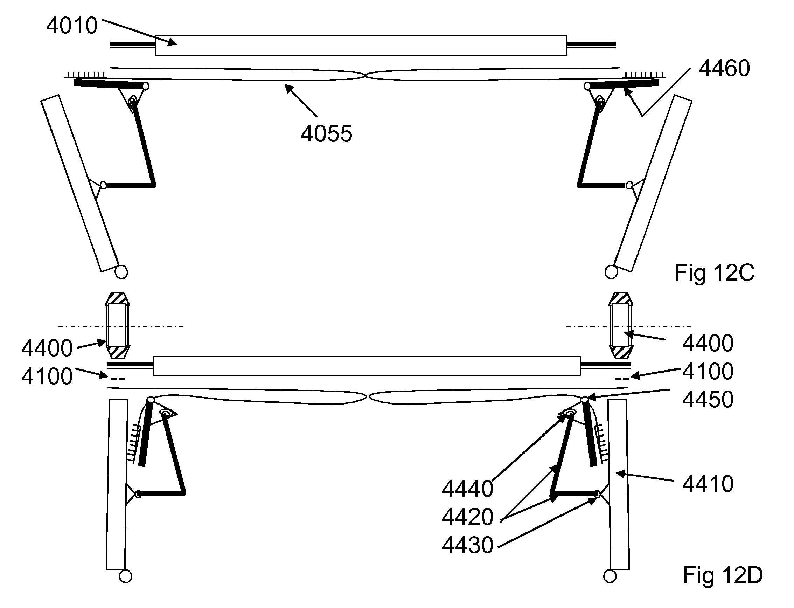

Yet a further variant of this embodiment is depicted in 12C, showing the centre piece and side panels essentially arranged as in Fig 12A. Instead of the laterally movable separation means, this variant comprises a folding means 4400 comprising a main lever 4410 acting via secondary lever 4420 and respective pivoting points 4430, 4440, and 4450 on a support means 4460. In Fig. 12C, this support means is shown to support the second longitudinally extending margin of the side panel in an essentially flat position relative to the rest of the side panel. In Fig. 12D, the levers are actuated, such that the support means 4460 is folded downwardly and the main lever 4410 is positioned such that it may act as a counteracting anvil to the connecting means 4400.

In a particular execution of a first and a second absorbent element being positioned onto a carrier web, such as a nonwoven web, which may be suitable as topsheet, the first and the second element may be cut and spaced off a single roll of absorbent material. Alternatively, they may come from two different rolls, and may have different composition and/or dimensions. In a further alternative, one or both of the elements may be formed "on-line", such as by a conventional core forming apparatus. The two elements may be fed to a combining unit in the spaced relationship. In a particular execution, the two elements may be positioned onto two web supports, which move at differential speed, such that the leading edge of the slightly faster moving trailing element overlaps the trailing edge of the slightly slower leading element, such that the two elements are delivered to the other elements of the articles in an overlapping (or shingled) positioning. The carrier web between the elements has then been folded in the longitudinal direction in a Z-folded manner. If the two elements are placed in the "detached topsheet" design as described in the above, and one of the elements is connected to the base in the crotch region whilst the other one is lifted, the carrier may form the opening and may be adapted to allow passage of liquids, like urine of faeces to penetrate through, such as by longitudinally extending slits applied to the carrier web between the elements. The cross-directionally extending edges of the elements may be straight in the cross-direction, but they may have varying shapes, such as rounded, or angled, or chevron like. A skilled person will readily realize that also the trailing edge of the leading element may overlie the leading edge of the trailing element.

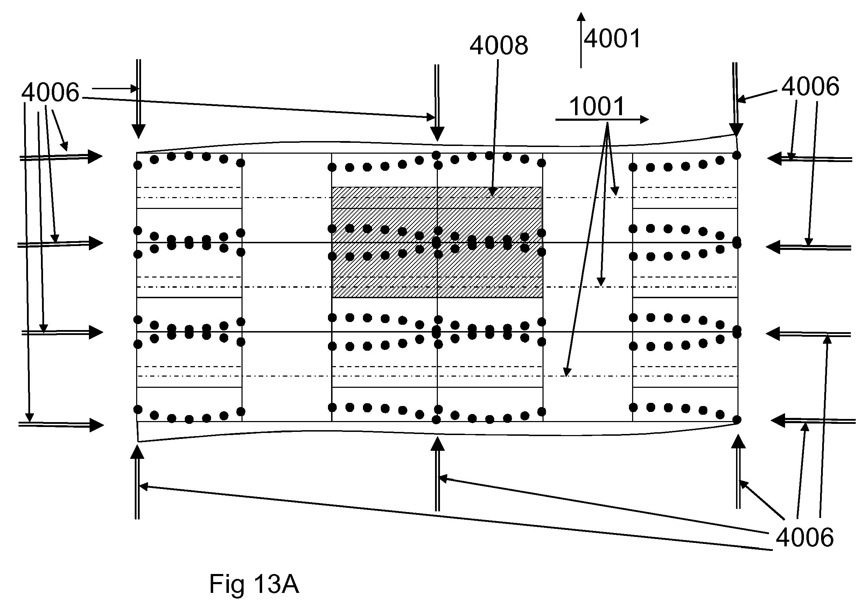

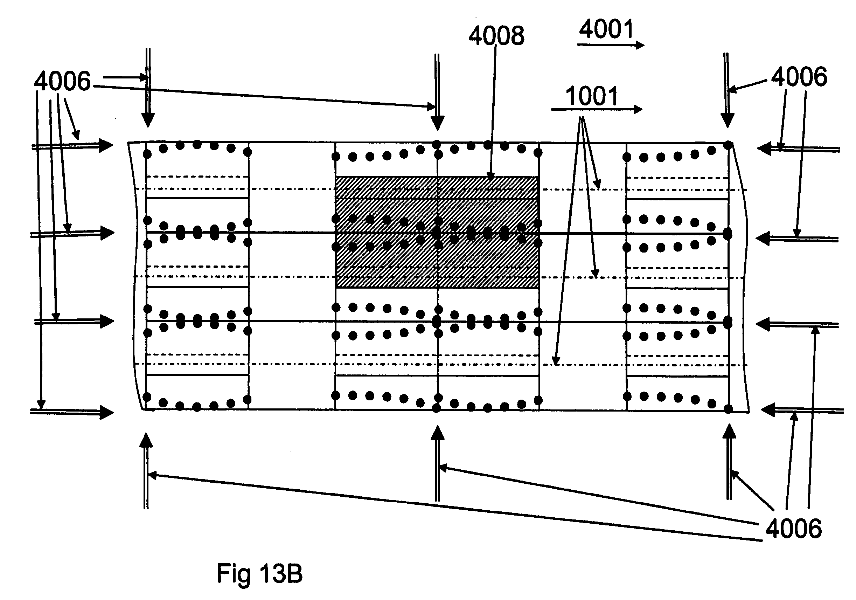

Fig. 13A and B schematically depict the concept of multi-lane converting. Therein, several products as schematically shown in Fig. 10 (although now with curved connecting lines 1123) are arranged adjacently during the production, and may form a single web, travelling at a machine direction 4001. The individual articles can be separated from this web, e.g. by cutting along the demarcation lines as indicated by the article demarcation arrows 4006. As shown in Fig. 13A, the longitudinal orientation of the centreline respectively of the final article 1001 may be perpendicular to the machine direction 4001, such that the overall web width corresponds to a multiple of the product length. As shown in Fig. 13B, the longitudinal orientation of the centre line respectively of the final article 1001 may be parallel to or aligned with the machine direction 4001, such that the overall web width corresponds to a multiple of the product centre piece width. In either case, one side panel strip 4008 (indicated by the shaded area), which is not positioned at the overall web margin, can contribute side panels to four neighbouring articles.

## Claims

1. An article for being worn on the lower torso of a wearer, said article comprising
a front region, a rear region and a crotch region there between, thereby defining a longitudinal (x-) and width (y-) direction and a longitudinally extending centre line of the article;
a centre strip extending from the front region through the crotch region to the rear region and exhibiting a first and a second longitudinally extending side margin and a first and a second surface;
at least a first and a second rear, optionally a first and a second front side panel connected to said centre piece in connecting regions, respectively,
wherein each of said side panels exhibit a first and second longitudinally extending side margin and a first and a second surface,
wherein
said centre piece and said sidepanels are adapted to form a waist hoop during use, and
said centre piece connecting regions extend essentially along said side margins of said centre piece in the rear region and optionally front region of said centre piece;
said side panel connecting regions extend essentially along said first side margins of said side panels,
said article being **characterized in that**
in a connecting region, at least in the rear region and optionally in the front region of said article, said second surfaces of said centre piece and said side panel are connected to themselves or to each other and **in that**
said first side margin of said first rear side panel is connected to said first side margin of said centre piece and said first side margin of said second rear side panel is connected to said second side margin of said centre piece;
optionally said first side margin of said first front side panel is connected to said first side margin of said centre piece and said first side margin of said second front side panel is connected to said second side margin of said centre piece.

2. An article according to claim 1, wherein said side panel connecting regions are formed by curved attachment lines or by predetermined irregularly shaped attachment regions.

3. An article according to claim 1 or 2, wherein said side panels comprise longitudinally extending second side margins opposite to said first margins, which are essentially unattached to said centre piece, at least in an in-use configuration.

4. An article according to any of claims 1 to 3, wherein
each of said second side margins of said first and second rear and optionally front side panels extend towards and optionally beyond said longitudinally extending centre line of said centre piece, but not to the connecting region of the respective opposite side panel.

5. An article according to any of claims 1 to 4, said article being
further **characterized in that**
said laterally extending side panel regions and said centre strip are double pleated (z-folded) along fold lines which are predominantly longitudinally oriented and which are essentially symmetrically to a longitudinally extending centre line of said article;
and **in that** said double pleat fold is permanently attached at least in one of the pleats by a first attachment at least in one of the hip, waist or hoop regions of the article.

6. An article according to any of claims 1 to 5 comprising a topsheet adapted for being in contact with the wearer during use, which is detachably overlying said centre piece at least in a portion of said longitudinal centre line in the crotch region whilst being attached to said centre strip in at least a further portion of said longitudinal centre line in the crotch region forwardly or rearwardly off set of said detached portion

7. An article according to any of the preceding claims, further comprising
a backsheet forming the surface of the article opposite to said topsheet
and a first absorbent element directly or indirectly attached to said backsheet at least in one of the front or back portion and a second absorbent element attached to said topsheet at least in a part of the portion, which is detachably overlying at least a part of said centre piece.

8. An article according to any of the preceding claims selected from the group of disposable and non-disposable absorbent articles, diapers, adult incontinence articles, training pants, menstrual pants, and underpants with limited washability.

9. A method for forming an article according to any of claims 1 to 8,
said article comprising a front waist region, a rear waist region and a crotch region there between, thereby defining a longitudinal (x-) and width (y-) article direction said method comprising the steps of
(a) providing one or more webs for forming a centre strip and side panels extending laterally outwardly at least in one of the front and rear region,
whereby said centre strip and said side panels are provided
(i) as separate webs or
(ii) as a unitary web;
(b) forming a pair of double pleat regions by folding at least one of the web(s), said fold lines extending essentially parallel and symmetrical to the longitudinal centreline of the article;
(c) affixing said pleat by attaching a first of said double pleat folds in the crotch region of the article;
(d) attaching said side panels and said centre piece if these are provided as separate webs.

10. A method according to claim 9, wherein said affixing in step (c) and said attaching in step (d) is executed by curved attachment lines or by predetermined irregularly shaped attachment regions.

11. A method for forming article according to any of claims 1 to 8,
said article comprising a front waist region, a rear waist region and a crotch region there between, thereby defining a longitudinal (x-) and width (y-) article direction and a longitudinal centre line and a first and a second rear and optionally a first and a second front side panel connected to said centre piece in respective regions said method comprising the steps of
(a) providing an essentially continuous centre strip web exhibiting a first and a second centre piece margin corresponding to the longitudinally extending side margins of the article and a centre piece centre line aligned with the longitudinal centre line of the article;
(b) providing one or more side panel webs for forming said side panels, each exhibiting a first and a second side panel margin corresponding to the longitudinally extending side panel margins of the article (c) separating individual side panel strips from said side panel webs;
(d) positioning
d)1)i) a first of said side panels such that its first side margin is essentially aligned with said first side margin of said centre piece in said rear region and such that its second side margin is oriented towards and optionally beyond said centre piece centre line, but not to the connecting region of the respective opposite side panel;
d)2)i) a second of said side panels such that its first side margin is essentially aligned with said second side margin of said centre piece in said rear region and such that its second side margin is oriented towards and optionally beyond said centre piece centre line, but not to the connecting region of the respective opposite side panel;
d)1)ii) optionally a third of said side panels such that its first side margin is essentially aligned with said first side margin of said centre piece in said front region and such that its second side margin is oriented towards and optionally beyond said longitudinal centre piece centre line, but not to the connecting region of the respective opposite side panel; d)2)ii) optionally a fourth of said side panels such that its first side margin is essentially aligned with said second side margin of said centre piece in said front region and such that its second side margin is oriented towards and optionally beyond said centre piece centre line, but not to the connecting region of the respective opposite side panel;
e) connecting said side panels to said centre piece in said connecting regions.

12. A method for forming an article according to claim 11, wherein said connecting regions are curved attachment lines or irregularly shaped attachment regions.

13. A method for forming an article according to claim 11 or 12, wherein said side panels are in a folded configuration.

14. A process for the manufacture of articles according to any of claims 11 to 13, wherein
said centre piece web is provided at a manufacturing direction width of several article centre piece widths or lengths.

15. A process for the manufacture of an article according to any of claims 11 to 14 wherein said article is a pants style article or a pre-closed article,
wherein said centre piece and said side panels each having a first and an opposite second surface distanced by the respective thickness of the web, such that during use both first surfaces are intended to be oriented in the same direction, such as towards the wearer;
and wherein the connecting being executed such that during the intended use of the article a waist hoop is formed, comprising said front and back regions of the centre piece and said side panels,
wherein said centre piece and said side panels are connected to each other in a first and a second connecting regions in a butt-type connection mode, such that in the connecting regions their respective second surfaces are oriented towards each other preferably by a melt or fusion bonding the process being an essentially continuous process with a process machine direction and a process cross-direction executed on a web handling equipment, preferably a turret,
wherein
said essentially continuous centre piece web has a centre piece web machine-direction corresponding essentially to the longitudinal direction of the centre piece in the article and defining a machine-directional centre line and a first and an opposite second machine-directionally extending side margin of the centre piece web, said centre piece
further has a first and a second centre piece connecting region in the proximity of the respective longitudinally extending side margins;
and a first and an opposite centre piece web surface,
said first and second essentially continuous side panel web
each have a sidepanel web cross-direction corresponding to a width direction of the side panel in the article and each having a first and an opposite second machine-directionally extending side margin ;
and each having a first and an opposite side panel web surface
said process further comprising the steps of
f) positioning said side panel webs relative to said centre piece web on said web handling equipment such that
- said first longitudinally extending side margin of said centre piece web is generally aligned with said first longitudinally extending side margin of said first side panel web; and
- said second longitudinally extending side margin of said centre piece web is generally aligned with said first longitudinally extending side margin of said second side panel web; and
- the second surfaces of said side panel webs are oriented towards said second surface of said centre piece web; and
- said second longitudinal extending side margin of said first side panel extends laterally outwardly of said first longitudinally extending side margin of said centre piece; and
- said second longitudinally extending side margin of said second side panel web extends laterally outwardly of said second longitudinally extending side margin of said centre piece; and
- such that the first connecting region comprises a layer of said centre piece web and two layers of said first side panel web and the second connecting region comprises a layer of said centre piece web and two layers of said second side panel web.
g) introducing a means for separating said first surfaces of said side panel webs in the connecting regions;
h) connecting said centre piece to the portion of said side panel webs with their second surfaces oriented towards said second surface of said centre piece, without connecting said sidepanels to themselves
i) separating articles and removing said articles ,
optionally
further comprising one or more of the following process steps:
(m) providing a topsheet and attaching said topsheet to said centre piece whilst leaving it essentially unattached to said centre strip at least along a portion of said centre strip.
(n) folding the resulting composite along or parallel to a cross-directional centre-line relative to the product in use orientation;
(o) combining side panels to each other (side seam);
(p) applying closure tapes of the adhesive or mechanical fastener type;
(q) applying two or more side panels, optionally still connected to each other by a "perf'n pop" connecting line, adapted to separate said panels upon activation such as through mechanical pulling online or by a user;
(r) providing leg hoop materials comprising a front, a rear and a crotch part, positioning and connecting these such that said crotch part is aligned essentially parallel to the longitudinal centre line of the article;
(s) providing leg elastics contracting at least in the crotch area;
(t) treating parts of the major inner surfaces with lotions;
(u) connecting parts of overfolded side panels to each other.

## Patentansprüche

1. Ein Artikel (1000) zum Tragen im unteren Bereich des Torsos eines Trägers,
wobei der Artikel (1000) folgendes umfasst:
einen vorderen Bereich (1012), einen hinteren Bereich (1018) und einen Schrittbereich (1015) dazwischen,
wobei eine Längs- (x-) (1001) und Breiten- (y-) (1002) Richtung bestimmt wird sowie eine in Längsrichtung des Artikels verlaufende Mittellinie,
einem Mittelstück (1010), das sich von dem vorderen Bereich (1012) durch den Schrittbereich (1015) zu dem hinteren Bereich (1018) erstreckt und das einen ersten und einen zweiten in Längsrichtung verlaufenden Seitenrand sowie eine erste und eine zweite Hauptoberfläche aufweist;
mindestens ein erstes und ein zweites hinteres, gegebenenfalls ein erstes und ein zweites vorderes, Seitenteil (1055), die jeweils mit dem Mittelstück (1010) in Verbindungsbereichen verbunden sind,
wobei jedes dieser Seitenteile (1055) einen ersten und einen zweiten in Längsrichtung verlaufenden Seitenrand und eine erste und eine zweite Hauptoberfläche aufweist,
worin
das Mittelstück (1010) und die Seitenteile (1055) geeignet sind, während der Benutzung einen Taillengürtel zu bilden,
und die Verbindungsbereiche des Mittelstücks sich im Wesentlichen entlang der Seitenränder des Mittelstücks im hinteren Bereich (1018) und gegebenenfalls im vorderen Bereich des Mittelstücks (1010) erstrecken,
und die Verbindungsbereiche der Seitenteile sich im Wesentlichen entlang der ersten Seitenränder der Seitenteile erstrecken,
die ersten Hauptoberflächen des Mittelstücks und der Seitenteile in einer Benutzungsanordnung zum Benutzer hin ausgerichtet sind,
wobei der Artikel (1000) **dadurch gekennzeichnet** wird,
dass in einem Verbindungsbereich, zumindest in dem hinteren Bereich (1018) und gegebenenfalls im vorderen Bereich (1012) des Artikels, die zweiten Flächen des Mittelstücks (1010) und der Seitenteile (1055) jeweils mit sich selbst oder miteinander verbunden sind, und
dass der erste Seitenrand des ersten hinteren Seitenteils mit dem ersten Seitenrand des Mittelstück verbunden ist
und der erste Seitenrand des zweiten hinteren Seitenteils mit dem zweiten Seitenrand des Mittelstücks verbunden ist;
gegebenenfalls der erste Seitenrand des ersten vorderen Seitenteils mit dem ersten Seitenrand des Mittelstücks verbunden ist, und der erste Seitenrand des zweiten vorderen Seitenteils mit dem zweiten Seitenrand des Mittelstücks verbunden ist.

2. Ein Artikel (1000) gemäß Anspruch 1, wobei die Verbindungsbereiche der Seitenteile gekrümmte Verbindungslinien oder vorgegebene unregelmäßig geformte Verbindungsbereiche sind.

3. Ein Artikel (1000) gemäß Anspruch 1 oder 2, wobei die Seitenteile (1055) jeweils zweite sich längs erstreckende Seitenränder haben, die gegenüber den ersten Seitenrändern positioniert sind und die im Wesentlichen zumindest in der Benutzungsanordnung nicht mit dem Mittelstück befestigt sind, umfassen.

4. Ein Artikel (1000) gemäß einem der Ansprüche 1 bis 3, wobei jeder der zweiten Seitenränder der ersten und zweiten hinteren und gegebenenfalls vorderen Seitenteile in Richtung der längs verlaufenden Mittellinie des Mittelstücks ragen, oder wahlweise darüber hinaus, jedoch nicht in den Verbindungsbereich des jeweiligen gegenüberliegenden Seitenteils.

5. Ein Artikel (1000) gemäß einem der Ansprüche 1 bis 4, wobei der Artikel ferner **dadurch gekennzeichnet ist,**
**dass** die seitlichen Bereiche der Seitenteile (1055) und des Mittelstücks (1010) doppelt gefaltet sind (Z- Faltung), entlang von Faltlinien, die überwiegend in Längsrichtung ausgerichtet sind und die im Wesentlichen symmetrisch zu einer in Längsrichtung verlaufenden Mittellinie des Artikels verlaufen, und
**dass** die Doppelfaltung wenigstens in einer der Falten dauerhaft fixiert ist durch eine erste Befestigung in zumindest einem der Hüft-, Taillen- oder Gürtelbereiche des Artikels.

6. Ein Artikel (1000) gemäß einem der Ansprüche 1 bis 5, umfassend eine Decklage (1025), die für den Kontakt mit dem Träger während des Gebrauchs geeignet ist, die dem Mittelstück (1010) lose aufliegt, zumindest in einem Abschnitt der genannten Längsmittellinie in dem Schrittbereich, während sie in mindestens einem weiteren Abschnitt der Längsmittellinie im Schrittbereich vor oder hinter dem lose aufliegenden Abschnitt mit dem Mittelstück verbunden ist.

7. Artikel (1000) gemäß einem der vorhergehenden Ansprüche, weiterhin umfassend eine Unterschicht (1045), die eine dem genannten Deckblatt (1025) gegenüberliegende Oberfläche des Artikels bildet,
und ein erstes Absorptionselement (1035), welches direkt oder indirekt mit der Unterschicht (1045) zumindest in einem der vorderen (1012) oder hinteren (1018) Bereiche verbunden ist,
und ein zweite Absorptionselement, welches mit der Decklage (1025) verbunden ist, zumindest in einem Teil des Abschnitts, der zumindest teilweise lose auf dem Mittelstück (1010) aufliegt.

8. Ein Artikel (1000) nach einem der vorhergehenden Ansprüche, ausgewählt aus der Gruppe von absorbierenden Einweg- und nicht-Einweg-Artikeln, Windeln, Inkontinenzartikel für Erwachsene, Trainingshöschen, Menstruationshöschen und Höschen mit begrenzter Waschbarkeit.

9. Ein Verfahren zur Herstellung eines Artikels (1000) gemäß einem der Ansprüche 1 bis 8,
wobei der Artikel (1000) einen vorderen Taillenbereich (1012), einen hinteren Taillenbereich (1018) und einen Schrittbereich (1015) aufweist, wodurch eine Längs- (x-) (1001) Richtung und eine Breiten-(y-) (1002) Richtung des Artikel bestimmt werden, und der Artikel weiterhin eine erste innenliegende Oberfläche und eine zweite, gegenüberliegende Oberfläche aufweist,
wobei das Verfahren die Schritte umfasst
(a) Bereitstellen eines oder mehrerer Bahnmaterialien zur Bildung eines Mittelstücks (1010) und von Seitenteilen (1055), die jeweils ein erste und eine zweite, gegenüberliegende Oberfläche aufweisen, wobei die ersten Oberflächen in einer Gebrauchsanordnung in Richtung eines Benutzers ausgerichtet sind, und die sich zumindest in einem der vorderen (1012) und hinteren (1018) Bereich seitlich erstrecken,
wobei das Mittelstück (1010) und die Seitenteile (1055) entweder
(i) als separate Bahnmaterialien oder
(ii) als einheitliches Bahnmaterial
bereitgestellt werden;
(b) Bilden eines Paares von Doppelfaltbereichen durch Falten zumindest einer der Bahn(en), wobei die zweiten Oberflächen der Bahnmaterialien zueinander ausgerichtet sind und wobei die Faltlinien sich im Wesentlichen parallel und symmetrisch zur Längsmittellinie (1005) des Artikels erstrecken;
(c) Fixierung der Falten durch Befestigen einer ersten Falte der Doppelfalten im Schrittbereich (1015) des Artikels;
(d) Befestigen der Seitenteile (1055) und des Mittelstücks (1010), wenn diese als separate Bahnmaterialien bereitgestellt werden.

10. Verfahren nach Anspruch 9, wobei das Fixieren in dem Schritt c) und das Befestigen in dem Schritt(d) durch gekrümmte Verbindungslinien oder vorgegebene unregelmäßig geformte Verbindungsbereiche erreicht wird.

11. Verfahren zum Herstellen eines Artikels (1000) nach einem der Ansprüche 1 bis 8,
wobei der Artikel einen vorderen Taillenbereich (1012), einen hinteren Taillenbereich (1018) und einem Schrittbereich (1015) dazwischen aufweist, wodurch eine Längs- (x-) Richtung (1001) und eine Breiten (y-) Richtung (1002) des Artikel definiert wird sowie eine Längsmittellinie (1005), sowie weiterhin ein erstes und ein zweites hinteres und wahlweise ein erstes und ein zweites vorderes Seitenteil (1055) ausweist, die mit dem Mittelstück (1010) in den entsprechenden Regionen verbunden sind,
wobei das Verfahren die Schritte umfasst
(a) Bereitstellen eines im wesentlichen kontinuierlichen Mittelstück-Bahnmaterials,
welches einen ersten und einen zweiten sich längs erstreckenden Mittelstück-Seitenrand aufweist, die den sich längs ersteckenden Seitenrändern des Artikels entsprechen, und
eine sich längs erstreckende Mittelstück-Mittellinie, die der Mittellinie des Artikels entspricht,
und welches eine erste Oberfläche aufweist, die in der Benutzungsanordnung in Richtung eines Benutzers ausgerichtet ist, und eine zweite, gegenüberliegende Oberfläche;
(b) Bereitstellen eines oder mehrerer Bahnmaterialien für die Bildung der Seitenteile (1055), die jeweils einen ersten und einen zweiten Seitenrand aufweisen, die den sich längs erstreckenden Seitenrändern des Artikels entsprechen;
(c) Abtrennen einzelner Seitenteile (1055) von dem Bahnmaterial für die Bildung der Seitenteile, die eine erste Oberfläche aufweisen, die in einer Benutzungsanordnung zum Benutzer hin ausgerichtet ist;
(d) Positionieren
d) 1) i) eines ersten Seitenteils (1055), so, dass sein erster Seitenrand im Wesentlichen ausgerichtet ist mit dem ersten Seitenrand des genannten Mittelstücks (1010) in dem hinteren Bereich (1018) und so, dass der zweite Seitenrand in Richtung der und gegebenenfalls über die genannte Mittellinie des Mittelstücks hinaus positioniert ist, aber nicht in den Verbindungsbereich des jeweilig gegenüberliegenden Seitenteils hineinreicht;
d) 2) i) eines zweiten Seitenteils (1055), so, dass sein erster Seitenrand im Wesentlichen ausgerichtet ist mit dem zweiten Seitenrand des genannten Mittelstücks (1010) in dem hinteren Bereich (1018) und so, dass der zweite Seitenrand der in Richtung und gegebenenfalls über die genannte Mittellinie des Mittelstücks hinaus positioniert ist, aber nicht in den Verbindungsbereich des jeweilig gegenüberliegenden Seitenteils hineinreicht;
d) 1) ii) gegebenenfalls eines dritten Seitenteils (1055), so, dass sein erster Seitenrand im Wesentlichen ausgerichtet ist mit dem ersten Seitenrand des genannten Mittelstücks (1010) in dem vorderen Bereich (1012) und so, dass der zweite Seitenrand in Richtung der und gegebenenfalls über die genannte Mittellinie des Mittelstücks hinaus positioniert ist, aber nicht in den Verbindungsbereich des jeweilig gegenüberliegenden Seitenteils hineinreicht;
d) 2) ii) gegebenenfalls eines vierten Seitenteils (1055), so, dass sein erster Seitenrand im Wesentlichen ausgerichtet ist mit dem zweiten Seitenrand des genannten Mittelstücks (1010) in dem vorderen Bereich (1012) und so, dass der zweite Seitenrand in Richtung der und gegebenenfalls über die genannte Mittellinie des Mittelstücks hinaus positioniert ist, aber nicht in den Verbindungsbereich des jeweilig gegenüberliegenden Seitenteils hineinreicht;
e) Verbinden der genannten Seitenteile (1055) mit dem Mittelstück (1010) in den Verbindungsbereichen,
wobei die Seitenteile (1055) und das Mittelstück (1010) so positioniert sind, dass die zweiten Oberflächen des Mittelstücks und der Seitenteile zueinander ausgerichtet sind

12. Ein Verfahren zur Bildung eines Artikels gemäß Anspruch 11, wobei die Verbindungsbereiche gekrümmte Verbindungslinien oder vorgegebene unregelmäßig geformte Verbindungsbereiche sind.

13. Ein Verfahren zur Bildung eines Artikels gemäß einem der Ansprüche 11 oder 12, wobei die Seitenteile (1055) in einer gefalteten Konfiguration sind.

14. Ein Verfahren zur Herstellung von Artikeln gemäß einem der Ansprüche 11 bis 13, wobei das Bahnmaterial zur Bildung des Mittelstücks mit einer Breite bereitgestellt wird, die ein Mehrfaches der Breite oder Länge des Mittelstücks beträgt.

15. Verfahren zur Herstellung von Artikeln (1000) gemäß einem der Ansprüche 11 bis 14,
wobei der Artikel (1000) ein hosenartiger Artikel oder ein vorabverschlossener Artikel ist,
wobei das Mittelstück (1010) und die Seitenteile (1055) jeweils eine erste und eine gegenüberliegende zweite Oberfläche aufweisen, die durch die jeweilige Dicke beabstandet sind, so, dass während der Verwendung die beiden ersten Oberflächen in die gleichen Richtung ausgerichtet sind, und zwar in Richtung des Trägers,
und wobei die Verbindung derartig ausgeführt ist, dass bei der bestimmungsgemäßen Verwendung des Artikels ein Taillengürtel gebildet wird, umfassend den vorderen (1012) und den hinteren (1018) Bereich des Mittelteils (1010) und die Seitenteile (1055),
wobei das Mittelstück (1010) und die Seitenteile (1055) miteinander in einem ersten und einem zweiten Verbindungsbereich durch eine Stumpfverbindung verbunden sind, vorzugsweise durch eine Schmelz- oder Schweißverbindung und derart, dass in den Verbindungsbereichen die jeweiligen zweiten Oberflächen einander zugewandt sind,
wobei das Verfahren ein im wesentlichen kontinuierliches Verfahren ist, mit einer Maschinenrichtung und einer Querrichtung und auf einem Bahnbearbeitungsapparat ausgeführt wird, vorzugsweise einer Trommel, und
wobei die im wesentlichen kontinuierliche Mittelstück-Materialbahn eine Maschinenrichtung aufweist, die im Wesentlichen der Längsrichtung des Mittelstücks des Artikels entspricht und dadurch eine Maschinenrichtungs-Mittelinie sowie einen ersten und einen zweiten, gegenüberliegenden Seitenrand des Mittelteils, die in der Maschinenrichtung verlaufen, definiert, wobei das Mittelstück
ferner einen ersten und einen zweiten Mittelstück-Verbindungsbereich aufweist, die sich in der Nähe der jeweiligen längsverlaufenden Seitenränder befinden,
und eine erste und eine zweite gegenüberliegende Mittelstück-Bahnoberfläche,
die erste und zweite im wesentlichen kontinuierliche Seitenteil-Materialbahn weisen
jeweils eine Seitenteilbahn-Querrichtung entsprechend der Breitenrichtung des Seitenteils in dem Artikel auf
sowie jeweils einen ersten und einen gegenüberliegenden zweiten Seitenrand in Maschinenrichtung
sowie jeweils eine erste und eine gegenüberliegende zweite Seitenteil-Materialbahnoberfläche,
wobei das Verfahren weiterhin die folgenden Schritte umfasst
f) Positionieren der Seitenteil-Materialbahn relativ zu der Mittelstück-Materialbahn auf dem Bahnbearbeitungsapparat, so, dass
- der erste in Längsrichtung verlaufende Seitenrand der genannten Mittelstück-Materialbahn im Wesentlichen entlang dem ersten in Längsrichtung verlaufenden Seitenrand der ersten Seitenteil-Materialbahn ausgerichtet wird, und
- der zweite in Längsrichtung verlaufende Seitenrand der genannten Mittelstück-Materialbahn im Allgemeinen mit dem ersten in Längsrichtung verlaufenden Seitenrand der zweiten Seitenteil-Materialbahn ausgerichtet wird, und
- die zweiten Oberflächen der genannten Seitenteil-Materialbahnen in die Richtung der zweiten Oberfläche der Mittelstück-Materialbahn ausgerichtet wird, und
- der zweite in Längsrichtung ausgerichtete Seitenrand des ersten Seitenteils erstreckt sich seitlich nach außen über den ersten in Längsrichtung verlaufenden Seitenrand des Mittelstück,
- der zweite in Längsrichtung verlaufende Seitenrand des zweiten Seitenteils erstreckt sich seitlich nach außen über den zweiten in Längsrichtung verlaufenden Seitenrand des Mittelstück, und
so, dass der erste Verbindungsbereich eine Schicht des Mittelstücks und zwei Schichten der ersten Seitenteils umfasst und
der zweite Verbindungsbereich eine Schicht des Mittelstücks und zwei Schichten des zweiten Seitenteils umfasst;
g) Einbringen einer Einrichtung zum Trennen der ersten Oberflächen der genannten Seitenwandstege in den Verbindungsbereichen;
h) Verbinden des Mittelstücks mit dem Teil der Seitenteil-Materialbahnen wobei deren zweite Oberflächen zu der zweiten Oberfläche des Mittelstücks ausgerichtet sind, ohne dass die Seitenteil-Materialbahnen miteinander verbunden werden;
i) Trennen der Artikel und das Entfernen der Artikel;
wobei das Verfahren wahlweise einen oder mehrere der folgenden Verfahrensschritte umfasst:
(m) Bereitstellen einer Decklage und Befestigen der Decklage an dem Mittelstück, wobei es zumindest entlang eines anderen Abschnitts des Mittelstücks im Wesentlichen unbefestigt bleibt;
(n) Falten des resultierenden Verbunds entlang oder parallel zu einer in Querrichtung verlaufenden Mittellinie relativ zu dem Produkt in einer Ausrichtung die der Benutzung entspricht;
(o) Verbinden der Seitenteile miteinander (Seitennaht);
(p) Anbringen von Verschlussbändern mit Klebstoff oder des mechanischen Typs;
(q) Anbringen von zwei oder mehr Seitenteilen, die wahlweise noch miteinander durch eine "perf'n pop" Verbindungslinie verbunden sind, die derart ausgeführt ist, dass sie durch Aktivierung getrennt werden kann, wie durch mechanischen Zug während der Herstellung oder durch einen Benutzer;
(r) Bereitstellen von Beinbündchen- Materialien, die eine vorderen Bereich, einen hinteren Bereich und einen Schrittbereich aufweisen, sowie das Positionieren und Verbinden dieser, so, dass der Schrittbereich im Wesentlichen parallel zu der Längsmittellinie des Artikels ausgerichtet ist;
(s) Bereitstellen elastischen Bändchen für Beinbündchen zumindest im Schrittbereich;
(t) Aufbringen von Lotionen auf einen wesentlichen Teil der inneren Flächen;
(u) Verbinden von um gefalteten Teilen der Seitenteile miteinander.

## Revendications

1. Article (1000) destiné à être porté sur le torse inférieur d'un porteur, ledit article comprenant
- une région avant (1012), une région arrière (1018) et une région d'entrejambe (1015) entre elles, définissant ainsi une direction longitudinale (x) (1001) et transversale (y) (1002) et une ligne des centres longitudinale (1005) de l'article;
- une bande centrale (1010) allant de la région avant (1012) à travers la région d'entrejambe (1015) de la partie arrière (1018) et présentant une première et une seconde marge latérale s'étendant longitudinalement et une première et une seconde surface majeure;
- au moins un premier et un deuxième panneau latéral (1055) arrière, éventuellement un premier et un deuxième panneau latéral avant, reliée à ladite bande centrale (1010) dans des régions de liaison, respectivement,
dans lequel chacun de lesdites panneaux latéraux (1055) présentent une première et une seconde marge s'étendent longitudinalement et une première et une seconde surface majeure,
dans lequel
ladite bande centrale (1010) et lesdits panneaux latéraux (1055) sont adaptés pour former une boucle de ceinture en utilisation, et
lesdites régions de liaison de la bande centrale s'entendent essentiellement le long de ladite marge latérale de ladite bande centrale dans la région arrière (1018) et éventuellement dans la région avant de ladite bande centrale (1010),
lesdites régions de liaison de lesdits panneaux latéraux s'étendent essentiellement le long de ladite première marge latérale de lesdites panneaux latéraux (1055),
dans lequel lesdites premières surfaces majeures de ladite bande centrale et de lesdites panneaux latéraux sont orientées à le porteur en utilisation,
ledit article (1000) étant **caractérisé en ce que**
dans une région de liaison, au moins dans la région arrière (1018) et éventuellement dans la région avant (1012) dudit article, lesdites surfaces secondaires de la bande centrale (1010) et des panneaux latéraux (1055) sont connectées à eux-mêmes ou les uns aux autres,
ladite première marge latérale dudit premier panneau latéral arrière est connectée à ladite première marge latérale de ladite bande centrale et ladite première marge latérale dudit deuxième panneau latéral arrière est connectée à ladite seconde marge latérale de ladite bande centrale;
éventuellement ladite première marge latérale dudit premier panneau latéral avant est connectée à ladite première marge latérale de ladite bande centrale et ladite première marge latérale dudit deuxième panneau latéral avant est connectée à ladite seconde marge latérale de ladite bande centrale;

2. Un article (1000) selon la revendication 1, dans lequel lesdites régions de liaison des lesdites panneaux latéraux sont formées par des lignes de liaison courbées ou par de régions de liaison ayant une forme irrégulière prédéterminées.

3. Un article (1000) selon la revendication 1 ou 2, dans lequel lesdits panneaux latéraux (1055) comprennent des marges latérales secondaire, s'étendant longitudinalement, opposée aux lesdites première marges latérales, qui sont essentiellement non attachée à ladite bande centrale (1010), au moins dans une configuration d'utilisation.

4. Un article (1000) selon l'une quelconque des revendications 1 à 3 dans lequel chacune desdites marges latérales secondaire de lesdites première at seconde panneau latéral arrière et éventuellement de lesdites première at seconde panneau latéral avant, étendent vers et éventuellement au-delà de ladite ligne des centres longitudinale (1005) de ladite bande centrale, mais pas à la région de liaison du panneau latéral opposé, respectivement.

5. Article (1000) selon l'une quelconque des revendications 1 à 4, ledit article étant **caractérisé en outre en ce que**
lesdites régions des panneaux latéraux (1055) s'étendent latéralement et ladite bande centrale (1010) sont plié à double (Z-pliée) le long de lignes de pliage qui sont orientées essentiellement longitudinalement et qui sont essentiellement symétrique par rapport à la ligne des centres longitudinale dudit article,
et **en ce que** ledit pli à double est fixée de manière permanente au moins dans l'un des plis par une première fixation dans au moins une des régions de la hanche, la taille ou boucle de l'article.

6. Article (1000) selon l'une quelconque des revendications 1 à 5, comprenant une feuille de dessus (1025) adaptée pour être en contact avec le porteur pendant l'utilisation, ce qui est sus-jacente de façon amovible de ladite bande centrale (1010) au moins dans une partie de ladite ligne des centres longitudinale (1005) dans la partie d'entrejambe tout en étant fixé à ladite bande centrale (1010) dans au moins une autre partie de ladite ligne des centres longitudinale (1005) dans la région d'entrejambe avant ou en arrière hors mis de ladite portion amovible.

7. Article (1000) selon l'une quelconque des revendications précédentes, comprenant en outre
une feuille de fond (1045) formant la surface de l'article opposée à ladite feuille de dessus (1025) et un premier élément absorbant (1035) attaché directement ou indirectement à ladite feuille de fond (1045) au moins dans une des régions avant (1012) ou arrière (1018) et un deuxième élément absorbant fixé à ladite feuille de dessus (1025) au moins dans la partie, qui est amovible au moins d'une partie de ladite bande centrale (1010).

8. Article selon l'une quelconque des revendications précédentes, choisis dans le groupe d'articles jetables et non jetables, des couches absorbantes, des articles pour l'incontinence des adultes, pantalons, pantalons de formation menstruelles, et slip avec lavabilité limitée.

9. Procédé pour la confection d'un article (1000) selon l'une quelconque des revendications 1 à 8,
ledit article (1000) comprenant une région de taille avant (1012), une région de taille arrière (1018) et une région d'entrejambe entre elles (1015), définissant ainsi un axe longitudinal (x) (1001) et un axe transversale (y) (1002) par rapport à l'article, l'article comprenant en plus une première surface intérieure et une seconde surface opposée,
ledit procédé comprenant les étapes consistant à
(a) fournir une ou plusieurs nappes des bandes pour former une bande centrale (1010) et des panneaux latéraux (1055) présentant une première et une seconde surface, dont les premières surfaces sont orientées vers le porteur pendant l'usage, et prolongeant latéralement vers l'extérieur au moins dans une région avant (1012) ou arrière (1018) et,
dans lequel ladite bande centrale (1010) et lesdits panneaux latéraux (1055) sont fournis
(i) en nappes de bande séparées ou
(ii) sous forme de bande unitaire;
(b) former une paire de régions doubles plis en pliant au moins une de les bandes, tel que lesdites secondes surfaces desdites bandes sont orientées à eux-mêmes, et lesdites lignes de pliage s'étendent essentiellement parallèles et symétrique par rapport à l'axe longitudinal de l'article (1005);
(c) fixer dudit pli en attachant un premier pli de ladite double pli dans la région d'entrejambe (1015) de l'article;
(d) attacher lesdits panneaux latéraux (1055) et ladite bande centrale (1010) si ceux-ci sont fournis à titre de nappes de bande séparées.

10. Procédé selon revendication 9, dans lequel la fixation dans l'étape (c) et l'attachement dans l'étape (d) sont exécuté(e) par des lignes de liaison courbées ou par de régions de liaison ayant une forme irrégulière prédéterminées.

11. Procédé pour la confection d'un article (1000) selon l'une quelconque des revendications 1 à 8,
ledit article comprenant une région de taille avant (1012), une région de taille arrière (1018) et une région d'entrejambe (1015) entre elles, définissant ainsi un axe longitudinal (x) (1001) et un axe latéral (y) (1002) par rapport à l'article et un axe central longitudinal (1005) et un première et un deuxième panneau latéral arrière, et éventuellement un premier et un deuxième panneau latéral avant, reliée à ladite bande centrale dans les régions respectives, ledit procédé comprenant les étapes consistant à
(a) fournir une nappe de bande centrale essentiellement continue présentant une première et une seconde marge du bande central, qui correspondent aux marges latérales qui s'étendent longitudinalement en l'article et un axe de bande centrale alignée avec la ligne des centres longitudinale de l'article (1005), présentant en plus une première surface orientée vers le porteur pendant l'utilisation et une seconde surface opposée ;
(b) fournir un ou plusieurs nappe(s) de bande pour former lesdites panneaux latéraux (1055), chacun présentant une première et une seconde marge latérale de panneau correspondant à l'extension longitudinale des marges de l'article ;
(c) séparer des panneaux latéraux (1055) de la nappe de bandes des panneaux latéraux, présentant en plus une première surface orientée vers le porteur pendant l'utilisation et une seconde surface opposée ;
(d) positionner
d)1)i) un premier desdits panneaux latéraux (1055) tel que sa première marge latérale est alignée essentiellement avec ladite première marge latérale de ladite bande centrale (1010) dans ladite région arrière (1018) et tel que sa seconde marge latérale est orientée vers et éventuellement au-delà de ladite ligne des centres longitudinale (1005) de l'article, mais pas à la région de liaison du panneau latéral opposée, respectivement;
d)2)i) un deuxième desdits panneaux latéraux (1055) tel que sa première marge latérale est alignée essentiellement avec ladite seconde marge latérale de ladite bande centrale (1010) dans ladite région arrière (1018) et tel que sa seconde marge latérale est orientée vers et éventuellement au-delà de ladite ligne des centres de l'article (1005), mais pas à la région de liaison du panneau latéral opposée, respectivement;
d)1)ii) éventuellement un troisième desdits panneaux latéraux (1055) tel que sa première marge latérale est alignée essentiellement avec ladite première marge latérale de ladite bande centrale (1010) dans ladite région avant (1012) et tel que sa seconde marge latérale est orientée vers et éventuellement au-delà de ladite ligne des centres longitudinale de l'article (1005), mais pas à la région de liaison du panneau latéral opposé respectif;
d 2) ii)) éventuellement un quatrième desdits panneaux latéraux (1055) tel que sa premier marge latérale est alignée essentiellement avec ladite deuxième marge latérale de ladite bande centrale (1010) dans ladite région avant (1012) et de telle sorte que sa seconde marge latérale est orientée vers et éventuellement au-delà de ladite ligne des centres longitudinale de l'article (1005), mais pas à la région de liaison du panneau latéral opposée respectivement;
e) reliant lesdits panneaux latéraux (1055) à ladite bande centrale (1010) dans lesdites régions de liaison, tel que dans l'étape d) lesdits panneaux latéraux (1055) et ladite bande centrale (1010) sont positionnées tel que les seconde surfaces des lesdits panneaux latéraux (1055) à ladite bande centrale (1010) sont orientées à eux-mêmes.

12. Procédé pour la confection d'un article (1000) selon revendication 11 tel que lesdites régions de liaison sont des lignes de liaison courbées ou des régions de liaison ayant une forme irrégulière.

13. Procédé pour la confection d'un article selon la revendication 11 ou 12, dans lequel lesdits panneaux latéraux (1055) sont dans une configuration pliée.

14. Procédé pour la confection d'un article selon l'une quelconque des revendications 11 à 13, dans lequel
ladite nappe de bande centrale essentiellement continue est prévue avec une largeur dans le sens travers de machine de plusieurs largeurs dans le sens longitudinale ou transversale du ladite bande centrale (1010).

15. Procédé pour la confection d'un article (1000) selon l'une quelconque des revendications 11 à 14, dans lequel ledit article (1000) est un article de type culotte ou d'un article pré-fermeture,
dans lequel ladite bande centrale (1010) et lesdits panneaux latéraux (1055) ayant chacun une première et une seconde surface opposée à distance par l'épaisseur respective, de telle sorte que pendant l'utilisation les deux premières surfaces sont destinées à être orientées dans la même direction, ce que vers le porteur,
et dans lequel la connexion étant exécutée de telle sorte que pendant l'utilisation prévue de l'article, une boucle de ceinture est formée, comprenant lesdites régions avant (1012) et arrière (1018) de la bande centrale (1010) et lesdits panneaux latéraux (1055), dans lequel ladite bande centrale (1010) et lesdits panneaux latéraux (1055) sont reliés les uns sur les autres dans une première et une deuxième région de liaison à un mode de connexion de type bout à bout, de telle sorte que dans les régions de liaison leurs secondes surfaces, respectivement, sont orientées les uns sur les autres, de préférence par une liaison de fonte ou fusion,
le procédé étant un procédé essentiellement continu avec une direction de la machine et une direction travers de machine, qui est exécuté sur un équipement de manipulation de bande, de préférence une tourelle,
dans lequel
ladite nappe essentiellement continu de bande centrale a une direction de sens machine correspondant essentiellement à la direction longitudinale de la bande centrale dans l'article, dont définissant pour la bande centrale une ligne des centres en sens machine et une première marge et une seconde marge opposée s'étendant en sens machine,
ladite bande centrale
comporte en outre une première et une deuxième région de liaison de bande centrale dans la proximité des marges latérales qui s'étendent longitudinalement,
et une première surface et une deuxième surface opposée de la bande centrale,
ladite ou lesdites nappe(s) de bande pour former lesdites panneaux latéraux (1055),
ayant une direction en sens de travers de machine correspondant à une direction de la largeur du panneau latéral dans l'article et ayant chacun une première marge et une seconde marge opposée en direction en sens de la machine; et
ayant chacun une première surface et une seconde surface opposée du panneau latéral,
dudit procédé comprenant en outre les étapes consistant à
f) positionner lesdites nappe(s) de bande pour former lesdites panneaux latéraux par rapport à ladite bande centrale sur ledit équipement de manipulation de bande tels que
- ladite première marge latérale en sens longitudinale de ladite nappe de la bande centrale est généralement alignée avec ladite première marge latérale en sens longitudinale de la nappe pour former le premier panneau latéral, et
- ladite seconde marge latérale en sens longitudinale de ladite nappe de la bande centrale est généralement alignée avec ladite première marge latérale en sens longitudinale de la nappe pour former le deuxième panneau latéral, et
- les secondes surfaces desdites nappes de bande des panneaux latéraux sont orientées en direction de ladite seconde surface de ladite bande centrale, et
- ladite seconde marge latérale s'étendant longitudinalement dudit premier panneau latéral se prolonge latéralement vers l'extérieur de ladite première marge latérale s'étendant longitudinalement de ladite bande centrale, et
- ladite seconde marge latérale s'étendant longitudinalement dudit deuxième panneau latéral se prolonge latéralement vers l'extérieur de ladite seconde marge latérale s'étendant longitudinalement de ladite bande centrale,
de telle sorte que la première région de liaison comprend une couche de ladite bande centrale et deux couches de la nappe pour former le premier panneau latéral et la deuxième région de liaison comprend une couche de ladite bande centrale et de deux couches la nappe pour former le deuxième panneau latéral ;
g) introduire un moyen pour séparer lesdits premier surfaces desdites nappes pour former les panneaux latéraux dans les régions de liaison;
h) relier ladite bande centrale avec les nappes pour former les panneaux latéraux avec leurs secondes surfaces orientées vers ladite seconde surface de ladite bande centrale, sans connecter lesdites panneaux latéraux à eux-mêmes ;
i) séparer et enlever lesdites articles,
éventuellement comprenant en outre une ou plusieurs des étapes suivantes :
(m) fournir une feuille de dessus et de fixer ladite feuille de dessus à ladite bande centrale tout en laissant au moins une partie de ladite bande centrale non-attachée à la bande centrale ;
(n) plier le composite résultant le long ou parallèlement à une ligne des centres dans la direction transversale par rapport au produit en utilisation;
(o) combiner des panneaux latéraux à l'un à l'autre (couture latérale);
(p) appliquer des bandes de fermeture de type adhésif ou mécanique ;
(q) appliquer au moins deux panneaux latéraux, éventuellement reliés entre eux par un ligne de liaison "perf'n pop", adapté pour séparer lesdits panneaux lors de l'activation tels que par traction mécanique pendant la fabrication ou par un utilisateur;
(r) fournir de matériel d'anneau de jambe comprenant une partie avant, une partie arrière et une partie entrejambe, et positionner et lier ledit matériel de telle sorte que ladite partie d'entrejambe est alignée essentiellement parallèle à la ligne des centres longitudinale de l'article;
(s) fournir des élastiques contractant au moins dans la partie d'entrejambe;
(t) traiter des portions des surfaces intérieures avec des lotions;
(u) lier des parties de panneaux latéraux pliés à eux-mêmes.
